# FASCICULE DE BREVET EUROPEEN

(11) **EP 0 876 362 B1**
(45) Date de publication et mention de la délivrance du brevet: **24.09.2003**
(21) Numéro de dépôt: 96942433.2
(22) Date de dépôt: 19.12.1996
(51) Int. Cl.: C07D 305/14, C07D 493/08, A61K 31/335

(54) **NOUVEAUX TAXOIDES, LEUR PREPARATION ET LES COMPOSITIONS PHARMACEUTIQUES QUI LES CONTIENNENT**
TAXAL DERIVATE, DEREN HERSTELLUNG UND DIESE ENTHALTENDE PHARMAZEUTISCHE ZUSAMMENSTELLUNGEN
NOVEL TAXOIDS, PREPARATION THEREOF AND PHARMACEUTICAL COMPOSITIONS CONTAINING SAME

(30) Priorité: 22.12.1995 FR 9515379
(43) Date de publication de la demande: 11.11.1998
(73) Titulaire: Aventis Pharma S.A., 92160 Antony (FR)
(72) Inventeur: BOUCHARD, Hervé, F-94320 Thiais (FR); COMMERCON, Alain, F-94400 Vitry-sur-Seine (FR)
(74) Mandataire: Le Pennec, Magali
(86) Numéro de dépôt international: FR9602031
(87) Numéro de publication internationale: WO97023473

(56) Documents cités:
- WO-A-93/21173

## Description

La présente invention concerne de nouveaux taxoïdes de formule générale : 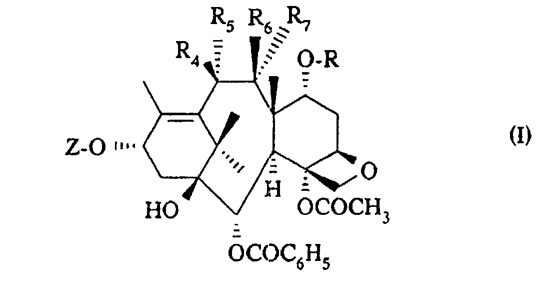 dans laquelle :
Z représente un atome d'hydrogène ou un radical de formule générale : 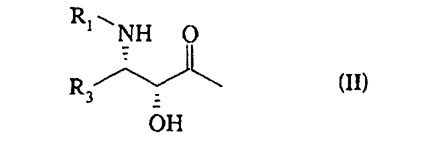 dans laquelle :
   R₁ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle ou un radical R₂-O-CO- dans lequel R₂ représente un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone, un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,- ou un radical hétérocyclyle saturé contenant 4 à 6 atomes de carbone éventuellement substitué par un ou plusieurs radicaux alcoyles contenant 1 à 4 atomes de carbone,
   R₃ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, cycloalcényles contenant 4 à 6 atomes de crabone, phényle ou α- ou β-naphtyle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonyl-amino, amino, alcoylamino, dialcoylamino, carboxy, alcoxycarbonyle, carbamoyle, alcoylcarbamoyle, dialcoylcarbamoyle, cyano, nitro et trifluorométhyle, ou un hétérocycle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles,
   ou bien R₄ représente un atome d'hydrogène,
   et R₆ et R₇ forment ensemble une fonction cétone, et
   et R et R₅ forment ensemble une liaison,
   ou bien R₄ représente un atome d'hydrogène ou un radical hydroxy ou un radical alcoxy contenant 1 à 6 atomes de carbone en chaîne droite ou ramifiée, alcényloxy contenant 3 à 6 atomes de carbone en chaîne droite ou ramifiée, alcynyloxy contenant 3 à 6 atomes de carbone en chaîne droite ou ramifiée, cycloalcoyloxy contenant 3 à 6 atomes de carbone, cycloalcényloxy contenant 3 à 6 atomes de carbone, alcanoyloxy dont la partie alcanoyle contient 1 à 6 atomes de carbone en chaîne droite ou ramifiée, aroyloxy dont la partie aryle contient 6 à 10 atomes de carbone, alcénoyloxy dont la partie alcénoyle contient 3 à 6 atomes de carbone en chaîne droite ou ramifiée, alcynoyloxy dont la partie alcynoyle contient 3 à 6 atomes de carbone en chaîne droite ou ramifiée,cycloalcanoyloxy contenant 3 à 6 atomes de carbone, alcoxyacétyle dont la partie alcoyle contient 1 à 6 atomes de carbone en chaîne droite ou ramifiée, alcoylthioacétyle dont la partie alcoyle contient 1 à 6 atomes de carbone en chaîne droite ou ramifiée, alcoyloxycarbonyloxy dont la partie alcoyle contient 1 à 6 atomes de carbone en chaîne droite ou ramifiée, ces radicaux étant éventuellement substitués par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone, ou bien R₄ représente un radical carbamoyloxy, alcoylcarbamoyloxy dont la partie alcoyle contient 1 à 4 atomes de carbone, dialcoylcarbamoyloxy dont chaque partie alcoyle contient 1 à 4 atomes de carbone ou un radical benzoyloxy ou hétérocyclylcarbonyloxy dans lequel la partie hétérocyclique représente un hétérocycle aromatique à 5 ou 6 chaînons contenant un ou plusieurs hétéroatomes choisis parmi les atomes d'oxygène, de soufre ou d'azote,
   et R₅ représente un atome d'hydrogène,
   ou R₄ et R₅ forment ensemble une fonction cétone,
   et R₆ représente un atome d'hydrogène, et
   R et R₇ forment ensemble une liaison.

De préférence les radicaux aryles pouvant être représentés par R₃ sont des radicaux phényles ou α- ou β-naphtyles éventuellement substitués par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluoro-méthyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles.

De préférence les radicaux hétérocycliques pouvant être représentés par R₃ sont des radicaux hétérocycliques aromatiques ayant 5 chaînons et contenant un ou plusieurs atomes, identiques ou différents, choisis parmi les atomes d'azote, d'oxygène ou de soufre, éventuellement substitués par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

Plus particulièrement, la présente invention concerne les produits de formule générale (I) dans laquelle Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical alcoyle contenant 1 à 6 atomes de carbone, alcényle contenant 2 à 6 atomes de carbone, cycloalcoyle contenant 3 à 6 atomes de carbone, phényle éventuellement substitué par ou plusieurs atomes ou radicaux, identiques ou différents, choisis parmi les atomes d'halogène (fluor, chlore) et les radicaux alcoyles (méthyle), alcoxy (méthoxy), dialcoylamino (diméthylamino), acylamino (acétylamino), alcoxycarbonylamino (tert-butoxycarbonylamino) ou trifluorométhyle ou un radical furyle-2 ou -3, thiényle-2 ou -3 ou thiazolyle-2, -4 ou -5 et, ou bien R₄ représente un atome d'hydrogène, et R₆ et R₇ forment ensemble une fonction cétone et R et R₅ forment ensemble une liaison, ou bien R₄ représente un radical hydroxy, alcoxy contenant 1 à 6 atomes de carbone, alcanoyloxy contenant 1 à 6 atomes de carbone ou alcoxyacétyle dont la partie alcoyle contient 1 à 6 atomes de carbone, et R₅ représente un atome d'hydrogène, et R₆ représente un atome d'hydrogène et R et R₇ forment ensemble une liaison, ou bien R₄ et R₅ forment ensemble une fonction cétone, et R₆ représente un atome d'hydrogène, et R et R₇ forment ensemble une liaison.

Plus particulièrement encore, la présente invention concerne les produits de formule générale (I) dans laquelle Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyle-2, furyle-3, thiényle-2, thiényle-3, thiazolyle-2, thiazolyle-4 ou thiazolyle-5, ou bien R₄ représente un atome d'hydrogène, et R₆ et R₇ forment ensemble une fonction cétone et R et R₅ forment ensemble une liaison, ou bien R₄ représente un radical hydroxy ou un radical méthoxy, acétoxy , propanoyloxy ou méthoxyacétoxy, et R₅ représente un atome d'hydrogène, et R₆ représente un atome d'hydrogène et R et R₇ forment ensemble une liaison.

Les produits de formule générale (I) dans laquelle Z représente un radical de formule générale (II) présentent des propriétés antitumorales et antileucémiques remarquables.

Selon l'invention, les produits de formule générale (I) dans laquelle, ou bien R₄ représente un atome d'hydrogène, R₆ et R₇ forment ensemble une fonction cétone et R et R₅ forment ensemble une liaison, ou bien R₄ représente un radical hydroxy, R₅ représente un atome d'hydrogène, R₆ représente un atome d'hydrogène et R et R₇ forment ensemble une liaison, peuvent être obtenus par action d'un agent de réduction sur un produit de formule générale 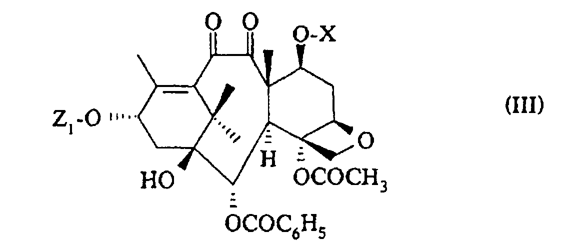 dans laquelle Z₁ représente un atome d'hydrogène ou un groupement protecteur de la fonction hydroxy ou un radical de formule générale : 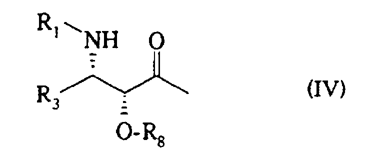 dans laquelle R₈ représente un groupement protecteur de la fonction hydroxy, et X représente, avec l'atome d'oxygène auquel il est lié, un groupe partant choisi parmi les radicaux alcoylsulfonyle contenant 1 à 4 atomes de carbone éventuellement substitué par un ou plusieurs atomes d'halogène, arylsulfonyle dont la partie aryle est un radical phényle éventuellement substitué par un ou plusieurs atomes ou radicaux, identiques ou différents, choisis parmi les atomes d'halogène et les radicaux alcoyle contenant 1 à 4 atomes de carbone, nitro ou trifluorométhyle, pour obtenir un produit de formule générale : 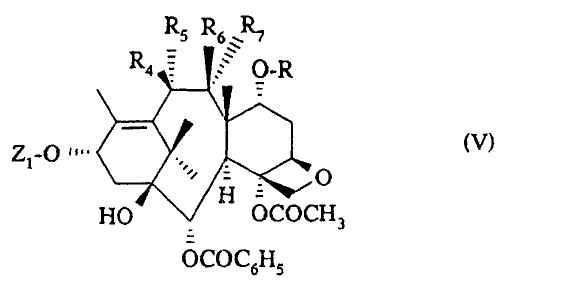 dans laquelle Z₁, R, R₄, R₅, R₆ et R₇ sont définis comme précédemment, suivi du remplacement du groupement protecteur représenté par Z₁ ou R₈ par un atome d'hydrogène.

Généralement, l'agent de réduction est choisi parmi les aluminohydrures ou les hydroborures tels que hydroborures de métal alcalin ou alcalino-terreux, comme l'hydroborure de sodium, en présence d'un alcool aliphatique contenant 1 à 4 atomes de carbone tel que l'éthanol, la réaction étant mise en oeuvre à une température comprise entre 0 et 50°C, de préférence voisine de 20°C.

De préférence le groupement protecteur représenté par R₈ est choisi parmi les groupements qui peuvent être facilement introduits et facilement éliminés sans toucher au reste de la molécule tels que les radicaux silylés comme le radical triéthylsilyle. Le remplacement du groupement protecteur, par un atome d'hydrogène, lorsqu'il représente un radical silylé, est généralement effectué au moyen d'un acide minéral tel que l'acide chlorhydrique dans un alcool aliphatique contenant 1 à 4 atomes de carbone à une température comprise entre -10 et 20°C, de préférence voisine de 0°C ou en présence d'une source d'ions fluorures tel qu'un complexe complexe acide fluorhydrique-triéthylamine en opérant dans un solvant organique inerte tel qu'un hydrocarbure aliphatique halogéné comme le dichlorométhane à une température comprise entre 0 et 50°C, de préférence voisine de 20°C.

La mise en oeuvre du procédé conduit généralement à un mélange d'un produit de formule générale (I) dans laquelle R₄ représente un atome d'hydrogène, R₆ et R₇ forment ensemble une fonction cétone, et R et R₅ forment ensemble une liaison, et d'un produit de formule générale (I) dans laquelle R₄ représente radical hydroxy, R₅ représente un atome d'hydrogène, R₆ représente un atome d'hydrogène, et R et R₇ forment ensemble une liaison qui sont séparés par les méthodes habituelles telles que la chromatographie.

Le produit de formule générale (III) peut être obtenu par action d'un agent d'oxydation sur un produit de formule générale : 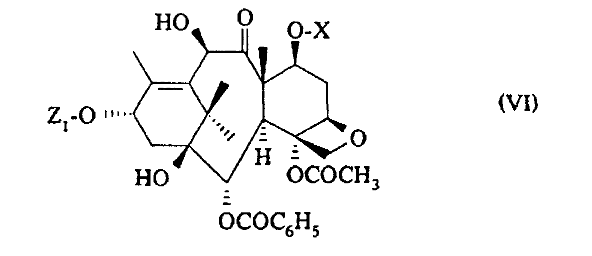 dans laquelle Z₁ et X sont définis comme précédemment.

Généralement l'agent d'oxydation est choisi parmi les agents qui permettent d'oxyder la fonction alcool secondaire sans toucher au reste de la molécule comme par exemple l'oxygène, le peruthénate d'ammonium, le bioxyde de manganèse, l'acétate de cuivre ou le chlorochromate de pyridinium. De préférence, on utilise le chlorochromate de pyridinium en opérant dans un solvant organique tel que les hydrocarbures aliphatiques éventuellement halogénés comme le dichlorométhane à une température comprise entre 0 et 50°C, de préférence voisine de 25°C.

Le produit de formule générale (VI) dans laquelle Z₁ et X sont définis comme précédemment peuvent être obtenus par action d'un halogénure de sulfonyle sur un produit de formule générale : 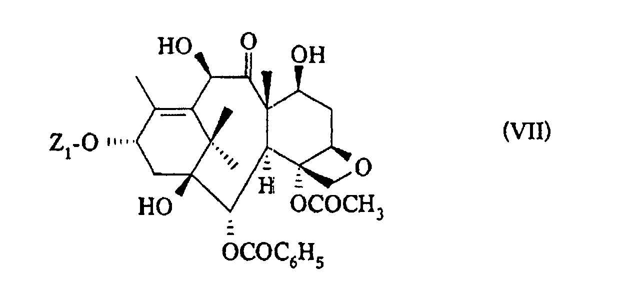 dans laquelle Z₁ est défini comme précédemment.

Le produit de formule générale (VI) dans laquelle X représente, de préférence, un radical trifluorométhylsulfonyle peut être obtenu par action d'un dérivé de l'acide trifluorométhanesulfonique tel que l'anhydride ou le N-phényl trifluorométhanesulfonimide dans un solvant organique inerte tel qu'un hydrocarbure aliphatique éventuellement halogéné comme le dichlorométhane en opérant en présence d'une base organique telle que la pyridine ou une amine aliphatique tertiaire comme la triéthylamine à une température comprise entre -50 et 20°C sur un produit de formule générale (VII).

Le produit de formule générale (VII) dans laquelle Z₁ représente un radical de formule générale (IV) dans laquelle R₈ est défini comme précédemment, peut être obtenu par action d'un agent de silylation sur un produit de formule générale : 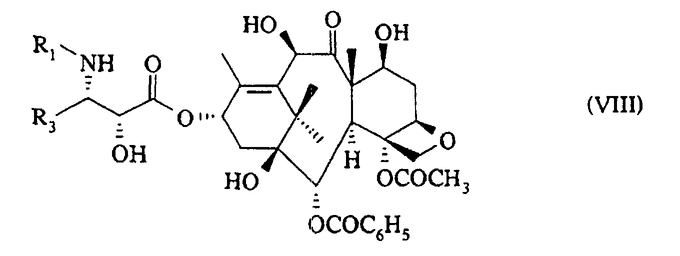 dans laquelle R₁ et R₃ sont définis comme précédemment.

Généralement, on utilise un halogénure de trialcoylsilyle tel que le chlorure de triéthylsilyle en opérant dans un hydrocarbure éventuellement halogéné tel que le dichlorométhane en présence d'une base organique telle que la pyridine ou une amine aliphatique tertiaire telle que la triéthylamine.

Le produit de formule générale (VIII) pour lequel R₃ représente un radical phényle et R₁ représente un radical tert-butyle est connu sous le nom de docetaxel. Les dérivés du docetaxel qui correspondent à la formule générale (VIII) peuvent être obtenus dans les conditions décrites dans les demandes internationales PCT WO 92/09589, WO 93/16060 et WO 94/12484.

Le produit de formule générale (VII) dans laquelle Z₁ représente un atome d'hydrogène est la 10-désacétylbaccatine III qui est extraite de manière connue à partir des feuilles d'if (Taxus baccata).

Selon l'invention les produits de formule générale (I) dans laquelle Z est défini comme précédemment, R₄ représente un radical alcoxy contenant 1 à 6 atomes de carbone en chaîne droite ou ramifiée, alcényloxy contenant 3 à 6 atomes de carbone en chaîne droite ou ramifiée, alcynyloxy contenant 3 à 6 atomes de carbone en chaîne droite ou ramifiée, cycloalcoyloxy contenant 3 à 6 atomes de carbone, cycloalcényloxy contenant 3 à 6 atomes de carbone, alcanoyloxy dont la partie alcanoyle contient 1 à 6 atomes de carbone en chaîne droite ou ramifiée, alcénoyloxy dont la partie alcénoyle contient 3 à 6 atomes de carbone en chaîne droite ou ramifiée, alcynoyloxy dont la partie alcynoyle contient 3 à 6 atomes de carbone en chaîne droite ou ramifiée, cycloalcanoyloxy contenant 1 à 6 atomes de carbone, alcoxyacétyle dont la partie alcoyle contient 1 à 6 atomes de carbone en chaîne droite ou ramifiée, alcoylthioacétyle dont la partie alcoyle contient 1 à 6 atomes de carbone en chaîne droite ou ramifiée, alcoyloxycarbonyloxy dont la partie alcoyle contient 1 à 6 atomes de carbone en chaîne droite ou ramifiée, ces radicaux étant éventuellement substitués par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone, ou bien R₄ représente un radical carbamoyloxy, alcoylcarbamoyloxy dont la partie alcoyle contient 1 à 4 atomes de carbone, dialcoylcarbamoyloxy dont chaque partie alcoyle contient 1 à 4 atomes de carbone ou un radical benzoyloxy ou hétérocyclylcarbonyloxy dans lequel la partie hétérocyclique représente un hétérocycle aromatique 5 ou 6 chaînons contenant un ou plusieurs hétéroatomes choisis parmi les atomes d'oxygène, de soufre ou d'azote, R₅ représente un atome d'hydrogène, R₆ représente un atome d'hydrogène, et R et R₇ forment ensemble une liaison, peuvent être obtenus par action d'un produit de formule générale :

R'₄-Y (IX)

dans laquelle R'₄ est tel que R'₄-O- est identique à R₄ défini comme précédemment et Y représente un groupe partant tel qu'un atome d'halogène, sur un produit de formule générale (V) dans laquelle Z₁ est défini comme précédemment, R₄ représente un radical hydroxy, R₆ représente un atome d'hydrogène et R et R₇ forment ensemble une liaison.

Généralement, l'action d'un produit de formule générale (IX) sur le produit de formule générale (V) défini ci-dessus est effectuée, après métallation éventuelle de la fonction hydroxy en 10 au moyen d'un hydrure de métal alcalin ou alcalino terreux tel que l'hydrure de sodium, d'un amidure de métal alcalin tel que le diisopropylamidure de lithium ou d'un alcoylure de métal alcalin tel que le n.butyllithium, en opérant dans un solvant organique tel que le diméthylformamide ou le tétrahydrofurane ou la pyridine à une température comprise entre 0 et 50°C, suivi éventuellement du remplacement du groupement protecteur de la fonction hydroxy Z₁ ou R₈ dans les conditions décrites précédemment.

Lorsque Z₁ est différent d'un radical de formule générale (IV), il est particulièrement avantageux d'effectuer la réaction sur un produit de formule générale (V) dans laquelle Z₁ représente un groupement protecteur de la fonction hydroxy qui est, de préférence, un radical triéthylsilyle. Dans ce cas, le groupement protecteur est introduit par action d'un halogénure de trialcoylsilyle, de préférence le chlorure de triéthylsilyle, sur un produit de formule générale (VI) dans laquelle Z₁ représente un atome d'hydrogène.

Selon l'invention, les produits de formule générale (I) dans laquelle Z représente un radical de formule générale (II), R₄ et R₅ forment ensemble une fonction cétone, R₆ représente un atome d'hydrogène, et R et R₇ forment ensemble une liaison, peuvent être obtenus par oxydation d'un produit de formule générale (V) dans laquelle Z₁ est défini comme précédemment, R₄ représente un radical hydroxy, R₅ représente un atome d'hydrogène, R₆ représente un atome d'hydrogène et R et R₇ forment ensemble une liaison, suivi éventuellement du remplacement du groupement protecteur représenté par Z₁ ou R₈ par un atome d'hydrogène dans les conditions décrites précédemment.

Généralement l'oxydation est effectuée dans les conditions décrites précédemment pour l'oxydation d'un produit de formule générale (VI).

Selon l'invention, les produits de formule générale (I) dans laquelle Z représente un radical de formule générale (II), R₄, R₅ et R₆ représentent chacun un atome d'hydrogène et R et R₇ forment ensemble une liaison, peuvent être obtenus à partir d'un produit de formule générale (V) dans laquelle Z₁ est défini comme précédemment, R₄ représente un radical hydroxy, R₅ et R₆ représentent chacun un atome d'hydrogène et R et R₇ forment ensemble une liaison, après transformation du radical hydroxy représenté par R₄ en dithiocarbonate suivie de la réduction du produit obtenu au moyen d'un hydrure de trialcoylétain tel que l'hydrure de tributylétain, suivi éventuellement du remplacement du groupement protecteur représenté par Z₁ ou R₈ par un atome d'hydrogène dans les conditions décrites précédemment.

Selon l'invention les produits de formule générale (I) peuvent aussi être obtenus par estérification d'un produit de formule générale (I) dans laquelle Z représente un atome d'hydrogène au moyen d'un acide de formule générale : 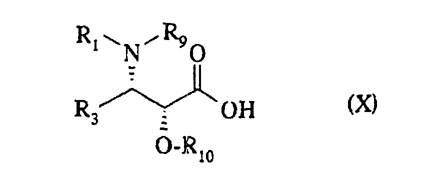 dans laquelle, ou bien R₉ représente un atome d'hydrogène et R₁₀ représente un groupement protecteur de la fonction hydroxy, ou bien R₉ et R₁₀ forment ensemble un hétérocycle saturé à 5 ou 6 chaînons, ou d'un dérivé de cet acide, suivi du remplacement des groupements protecteurs par des atomes d'hydrogène.

Les conditions d'estérification et de remplacement des groupements protecteurs sont identiques à celles qui sont décrites, par exemple, dans les demandes internationales PCT WO 92/09589, WO 93/16060 et WO 94/12484.

Les nouveaux produits de formule générale (I) obtenus par la mise en oeuvre des procédés selon l'invention peuvent être purifiés selon les méthodes connues telles que la cristallisation ou la chromatographie.

Les produits de formule générale (I) dans laquelle Z représente un radical de formule générale (II) présentent des propriétés biologiques remarquables.

In vitro, la mesure de l'activité biologique est effectuée sur la tubuline extraite de cerveau de porc par la méthode de M.L. Shelanski et coll., Proc. Natl. Acad. Sci. USA, 70, 765-768 (1973). L'étude de la dépolymérisation des microtubules en tubuline est effectuée selon la méthode de G. Chauvière et coll., C.R. Acad. Sci., 293, série II, 501-503 (1981). Dans cette étude les produits de formule générale (I) dans laquelle Z représente un radical de formule générale (II) se sont montrés au moins aussi actifs que le Taxol et le Taxotère.

In vivo, les produits de formule générale (I) dans laquelle Z représente un radical de formule générale (II) se sont montrés actifs chez la souris greffée par le mélanome B16 à des doses comprises entre 1 et 10 mg/kg par voie intrapéritonéale, ainsi que sur d'autres tumeurs liquides ou solides.

Les nouveaux produits ont des propriétés anti-tumorales et plus particulièrement une activité sur les tumeurs qui sont résistantes au Taxol® ou au Taxotère®. De telles tumeurs comprennent les tumeurs du colon qui ont une expression élevée du gène mdr 1 (gène de la multi-drug resistance). La multi-drug resistance est un terme habituel se rapportant à la résistance d'une tumeur à différents produits de structures et de mécanismes d'action différents. Les taxoïdes sont généralement connus pour être fortement reconnus par des tumeurs expérimentales telles que P388/DOX, une lignée cellulaire sélectionnée pour sa résistance à la doxorubicine (DOX) qui exprime mdr 1.

Les exemples suivants illustrent la présente invention.

### EXEMPLE 1

A une solution de 0,65 g de tert-butoxycarbonylamino-3 phényl-3 triéthylsilyloxy-2 propionate-(2R,3S) d'acétoxy-4α benzoyloxy-2α époxy-5β,20 hydroxy-1β dioxo-9,10 trifluorométhanesulfonyloxy-7β taxène-11 yle-13α dans 6,5 cm3 d'éthanol absolu, maintenue sous atmosphère d'argon, à une température voisine de 20°C, on ajoute 117 mg de borohydrure de sodium. Après 5 minutes à une température voisine de 20°C, le mélange réactionnel est dilué avec 50 cm3 d'acétate d'éthyle. La phase organique est lavée avec 3 fois 10 cm3 d'eau distillée, puis 2 fois 10 cm3 d'une solution aqueuse saturée en chlorure de sodium, séchée sur sulfate de magnésium, filtrée sur verre fritté et concentrée sous pression réduite (2,7 kPa) à une température voisine de 40°C. On obtient ainsi 600 mg d'une meringue blanche que l'on combine avec 313 mg d'un même mélange brut obtenu à partir de 500 mg de tert-butoxycarbonylamino-3 phényl-3 triéthylsilyloxy-2 propionate-(2R,3S) d'acétoxy-4α benzoyloxy-2α époxy-5β,20 hydroxy-1β dioxo-9,10 trifluorométhanesulfonyloxy-7β taxène-11 yle-13α dans les mêmes conditions. La purification s'effectue par chromatographie à pression atmosphérique sur 100 g de silice (0,063-0,2 mm) contenus dans une colonne de 3,5 cm de diamètre en éluant avec un mélange acétate d'éthyle-dichlorométhane (gradient d'élution de 2-98 à 15-85 en volumes) en recueillant des fractions de 20 cm3. Les fractions ne contenant que les produits cherchés sont réunies et concentrées à sec sous pression réduite (2,7 kPa) à 40°C pendant 2 heures. On obtient ainsi 153 mg de tert-butoxycarbonylamino-3 phényl-3 triéthylsilyloxy-2 propionate-(2R,3S) d'acétoxy-4α benzoyloxy-2α époxy-5β,20 hydroxy-1β époxy-7α,10α oxo-9 taxène-11 yle-13α sous forme d'une meringue blanche et 384 mg de tert-butoxycarbonylamino-3 phényl-3 triéthylsilyloxy-2 propionate-(2R,3S) d'acétoxy-4α benzoyloxy-2α époxy-5β,20 dihydroxy-1β,10β époxy-7α,9α taxène-11 yle-13α sous forme d'une meringue blanche.

Le tert-butoxycarbonylamino-3 phényl-3 triéthylsilyloxy-2 propionate-(2R,3S) d'acétoxy-4α benzoyloxy-2α époxy-5β,20 hydroxy-1β époxy-7α,10α oxo-9 taxène-11 yle-13α présente les caractéristiques suivantes :
- spectre de R.M.N. ¹H (400 MHz ; CDCl₃ ; δ en ppm ; constantes de couplage J en Hz) : 0,34 et 0,41 (2 mts, 6H : CH₂ du triéthylsilyl en 2') ; 0,77 (t, J = 7,5, 9 H : CH₃ du triéthylsilyl en 2') ; 1,23 (s, 3H : CH₃) ; 1,38 (s, 3H : CH₃) ; 1,40 (s, 9H : C(CH₃)₃) ; 1,82 (s, 3H : CH₃) ; 1,90 (s, 3H : CH₃) ; 1,93 (s, 1H : OH en 1) ; de 2,15 à 2,40 (mt, 2H : CH₂ en 14) ; de 2,15 à 2,40 et 2,48 (2 mts, 1H chacun : CH₂ en 6) ; 2,48 (s, 3H : COCH₃) ; 3,70 (d, J = 8, 1H : H en 7) ; 4,25 et 4,32 (2 d, J = 8, 1H chacun : CH₂ en 20) ; 4,58 (d, J = 7, 1H : H en 3) ; 4,59 (s large, 1H : H en 2') ; 4,86 (mt, 1H : H en 10) ; 5,11 (d, J = 5, 1H : H en 5) ; 5,32 (d large, J = 10, 1H: H en 3') ; 5,56 (d, J = 10, 1H : CONH) ; 5,62 (d, J = 7, 1H : H en 2) ; 6,34 (t large, J = 9, 1H : H en 13) ; de 7,25 à 7,45 (mt, 5H : H aromatiques en 3') ; 7,50 (t, J = 7,5, 2H : OCOC₆H₅ H en méta) ; 7,62 (t, J = 7,5, 1H : OCOC₆H₅ H en para) ; 8,13 (d, J = 7,5, 2H : OCOC₆H₅ H en ortho).

Le tert-butoxycarbonylamino-3 phényl-3 triéthylsilyloxy-2 propionate-(2R,3S) d'acétoxy-4α benzoyloxy-2α époxy-5β,20 dihydroxy-1β,10β époxy-7α,9α taxène-11 yle-13α présente les caractéristiques suivantes :
- spectre de R.M.N. ¹H (400 MHz ; CDCl₃ ; δ en ppm ; constantes de couplage J en Hz) : 0,33 et 0,40 (2 mts, 6H : CH₂ du triéthylsilyl en 2') ; 0,75 (t, J = 7,5, 9 H : CH₃ du triéthylsylil en 2') ; 1,13 (s, 3H : CH₃) ; 1,27 (s, 3H : CH₃) ; 1,37 (s, 9H : C(CH₃)₃) ; 1,75 (s, 3H : CH₃) ; 1,85 (s, 1H : OH en 1) ; 2,04 (s, 3H : CH₃) ; 2,23 et de 2,30 à 2,50 (respectivement dd et mt, J = 15 et 8, 1H chacun : CH₂ en 14) ; de 2,30 à 2,50 (mt, 2H : CH₂ en 6) ; 2,48 (s, 3H : COCH₃) ; 2,55 (d, J = 7, 1H : OH en 10) ; 4,05 et 4,29 (2 d, J = 7,5, 1H chacun : CH₂ en 20) ; 4,17 (d, J = 6, 1H : H en 3) ; 4,60 (s large, 1H : H en 2') ; de 4,75 à 4,90 (mt, 3H : H en 7 - H en 9 et H en 10) ; 4,97 (s large, 1H : H en 5) ; 5,33 (d large, J = 10, 1H : H en 3') ; 5,54 (d, J = 10, 1H : CONH) ; 5,80 (d, J = 6, 1H : H en 2) ; 6,18 (t large, J = 8, 1H : H en 13) ; de 7,25 à 7,45 (mt, 5H : H aromatiques en 3') ; 7,49 (t, J = 7,5, 2H : OCOC₆H₅ H en méta) ; 7,62 (t, J = 7,5, 1H : OCOC₆H₅ H en para) ; 8,15 (d, J = 7,5, 2H : OCOC₆H₅ H en ortho).

Une solution de 126 mg de tert-butoxycarbonylamino-3 phényl-3 triéthylsilyloxy-2 propionate-(2R,3S) d'acétoxy-4α benzoyloxy-2α époxy-5β,20 dihydroxy-1β,10β époxy-7α,9α taxène-11 yle-13α dans 1,7 cm3 d'éthanol chlorhydrique 0,1N est agitée, sous atmosphère d'argon, à une température voisine de 0°C, pendant 1 heure. Le mélange réactionnel est alors dilué avec 20 cm3 de dichlorométhane. La phase organique est lavée avec 2 fois 5 cm3 d'eau distillée, puis avec 2 fois 5 cm3 d'une solution aqueuse saturée en chlorure de sodium, séchée sur sulfate de magnésium, filtrée sur verre fritté et concentrée sous pression réduite (2,7 kPa) à une température voisine de 40°C. On obtient ainsi 130 mg d'une meringue ivoire que l'on purifie par chromatographie préparative sur couche mince [12 plaques préparatives Merck, Kieselgel 60F254 : 20x20 cm ; épaisseur 0,25 mm ; dépôt en solution dans le dichlorométhane] en éluant 2 fois par un mélange méthanol-dichlorométhane (5-95 en volumes). Après élution de la zone correspondant au produit recherché par un mélange méthanol-dichlorométhane (15-85 en volumes), filtration sur verre fritté, puis évaporation des solvants sous pression réduite (2,7 kPa) à une température voisine de 40°C, on obtient 22,6 mg de tert-butoxycarbonylamino-3 phényl-3 hydroxy-2 propionate-(2R,3S) d'acétoxy-4α benzoyloxy-2α époxy-5β,20 dihydroxy-1β,10β époxy-7α,9α taxène-11 yle-13α sous forme de meringue blanche dont les caractéristiques sont les suivantes :
- spectre de R.M.N. ¹H (400 MHz ; CDCl₃ ; à une température de 333°K, δ en ppm ; constantes de couplage J en Hz) : 1.14 (s, 3H : CH₃) ; 1,25 (s, 3H : CH₃) ; 1,40 (s, 9H : C(CH₃)₃) ; 1,74 (s, 3H : CH₃) ; 1,86 (s, 1H : OH en 1) ; 1,95 (s, 3H : CH₃) ; de 2,15 à 2,45 (mt, 4H : CH₂ en 14 et CH₂ en 6) ; 2,33 (s, 3H : COCH₃) ; 2,50 (mf, 1H : OH en 10) ; 3,67 (mf, 1H : OH en 2') ; 4,06 et 4,27 (2 d, J = 7,5, 1H chacun : CH₂ en 20) ; 4,17 (d, J = 6, 1H : H en 3) ; 4,65 (mt, 1H : H en 2') ; de 4,75 à 4,90 (mt, 3H : H en 7 - H en 9 et H en 10) ; 4,93 (s large, 1H : H en 5) ; 5,30 (d large, J = 10, 1H : H en 3') ; 5,50 (d, J = 10, 1H CONH) ; 5,79 (d, J = 6, 1H : H en 2) ; 6,06 (t large, J = 9, 1H H en 13) ; 7,30 (t, J = 7,5, 1H H en para de l'aromatique en 3') ; 7,38 (t, J = 7,5, 2H : H en méta de l'aromatique en 3') ; 7,44 (d, J = 7, 2H : H en ortho de l'aromatique en 3') ; 7,49 (t, J = 7,5, 2H : OCOC₆H₅ H en méta) ; 7,61 (t, J = 7,5, 1H : OCOC₆H₅ H en para) ; 8,13 (d, J = 7,5, 2H : OCOC₆H₅ H en ortho).

Le tert-butoxycarbonylamino-3 phényl-3 triéthylsilyloxy-2 propionate-(2R,3S) d'acétoxy-4a benzoyloxy-2α époxy-5β,20 hydroxy-1β dioxo-9,10 trifluorométhanesulfonyloxy-7β taxène-11 yle-13α peut être préparé de la manière suivante :

A une suspension de 1,87 g de tert-butoxycarbonylamino-3 phényl-3 triéthylsilyloxy-2 propionate-(2R,3S) d'acétoxy-4α benzoyloxy-2α époxy-5β,20 dihydroxy-1β,10β oxo-9 trifluorométhanesulfonyloxy-7β taxène-11 yle-13α et de 4 g de tamis moléculaire 4 Å activé dans 10 cm3 de dichlorométhane anhydre, maintenue sous atmosphère d'argon, à une température voisine de 20°C, on ajoute rapidement 1,91 g de chlorochromate de pyridinium. Le mélange réactionnel est agité pendant 20 heures, à une température voisine de 20°C, puis purifié directement par dépôt sur une colonne de chromatographie à pression atmosphérique contenant 200 g de silice (0,063-0,2 mm ; colonne de 3,5 cm de diamètre) en éluant avec du dichlorométhane seul puis avec un mélange méthanol-dichlorométhane (0,5-99,5 en volumes) en recueillant des fractions de 15 cm3. Les fractions ne contenant que le produit cherché sont réunies et concentrées à sec sous pression réduite (2,7 kPa) à 40°C pendant 2 heures. On obtient ainsi 1,16 g de tert-butoxycarbonylamino-3 phényl-3 triéthylsilyloxy-2 propionate-(2R,3S) d'acétoxy-4α benzoyloxy-2α époxy-5β,20 hydroxy-1β dioxo-9,10 trifluorométhanesulfonyloxy-7β taxène-11 yle-13α sous forme d'une meringue jaune pâle dont les caractéristiques sont les suivantes :
- spectre de R.M.N. ¹H (400 MHz ; CDCl₃ ; δ en ppm ; constantes de couplage J en Hz) : 0,42 (mt, 6H : CH₂ du triéthylsilyl en 2') ; 0,81 (t, J = 7,5, 9 H : CH₃ du triéthylsilyl en 2') ; 1,26 (s, 3H : CH₃) ; 1.35 (s, 3H : CH₃) ; 1,37 (s, 9H : C(CH₃)₃) ; 1,93 (s, 3H : CH₃) ; 2,01 (s, 3H : CH₃) ; 2,23 et 2,43 (2 dd, J = 15 et 9, 1H chacun : CH₂ en 14) ; 2,36 et 2,89 (2 mt, 1H chacun : CH₂ en 6) ; 2,57 (s, 3H : COCH₃) ; 3,82 (d, J = 7, 1H : H en 3) ; 4,23 et 4,42 (2d, J = 8,5, 1H chacun : CH₂ en 20) ; 4,58 (s large, 1H : H en 2') ; 4,95 (d large, J = 9,5, 1H : H en 5) ; 5,28 (dd, J = 10 et 7,5, 1H : H en 7) ; 5,30 (d large, J = 10,1H : H en 3') ; 5,52 (d, J = 10, 1H : CONH) ; 5,87 (d, J = 7, 1H : H en 2) ; 6,28 (t large, J = 9,1H : H en 13) ; de 7,25 à 7,45 (mt, 5H : H aromatiques en 3') ; 7,55 (t, J = 7,5, 2H : OCOC₆H₅ H en méta) ; 7,67 (t, J = 7,5, 1H : OCOC₆H₅ H en para) ; 8,13 (d, J = 7,5 Hz, 2H : OCOC₆H₅ H en ortho).

Le tert-butoxycarbonylamino-3 phényl-3 triéthylsilyloxy-2 propionate-(2R,3S) d'acétoxy-4α benzoyloxy-2α époxy-5β,20 dihydroxy-1β,10β oxo-9 trifluorométhanesulfonyloxy-7β taxène-11 yle-13α peut être préparé de la manière suivante :

A une suspension de 8,85 g de tert-butoxycarbonylamino-3 phényl-3 triéthylsilyloxy-2 propionate-(2R,3S) d'acétoxy-4α benzoyloxy-2α époxy-5β,20 trihydroxy-1β,7β,10β oxo-9 taxène-11 yle-13α et 2 g de tamis moléculaire 4 Å activé dans 50 cm3 de dichlorométhane anhydre et 3,9 cm3 de pyridine anhydre, maintenue sous atmosphère d'argon, à une température voisine de -30°C, on ajoute goutte à goutte une solution de 3,2 cm3 d'anhydride trifluorométhanesulfonique dans 3 cm3 de dichlorométhane anhydre. Le mélange réactionnel est agité pendant 5 minutes à -35°C, puis pendant 1 heure à une température voisine de 0°C. Après refroidissement à une température voisine de -10°C, on ajoute 6 cm3 d'eau distillée. Après filtration sur verre fritté garni de célite, rinçage du verre fritté par 20 cm3 d'un mélange acétate d'éthyle-dichlorométhane (50-50 en volumes) et décantation, la phase organique est lavée par deux fois 10 cm3 d'eau distillée, séchée sur sulfate de magnésium, filtrée sur verre fritté et concentrée sous pression réduite (2,7 kPa) à une température voisine de 40°C. On obtient ainsi 11,3 g d'une meringue orangée que l'on purifie par chromatographie à pression atmosphérique sur 800 g de silice (0,063-0,2 mm) contenus dans une colonne de 7 cm de diamètre en éluant avec un mélange méthanol-dichlorométhane (1-99 puis 2-98 en volumes) en recueillant des fractions de 60 cm3. Les fractions ne contenant que le produit cherché sont réunies et concentrées à sec sous pression réduite (2,7 kPa) à 40°C pendant 2 heures. On obtient ainsi 9,55 g de ten-butoxycarbonylamino-3 phényl-3 triéthylsilyloxy-2 propionate-(2R,3S) d'acétoxy-4α benzoyloxy-2α époxy-5β,20 dihydroxy-1β,10β oxo-9 trifluorométhanesulfonyloxy-7β taxène-11 yle-13α en mélange. Ce mélange est purifié par chromatographie à pression atmosphérique sur 700 g de silice (0,063-0,2 mm) contenus dans une colonne de 6 cm de diamètre en éluant avec du dichlorométhane seul, puis avec un mélange acétate d'éthyle-dichlorométhane (5-95 en volumes) en recueillant des fractions de 60 cm3. Les fractions ne contenant que le produit cherché sont réunies et concentrées à sec sous pression réduite (0,27 kPa) à 40°C pendant 2 heures. On obtient ainsi 4,09 g de tert-butoxycarbonylamino-3 phényl-3 triéthylsilyloxy-2 propionate-(2R,3S) d'acétoxy-4α benzoyloxy-2α époxy-5β,20 dihydroxy-1β,10β oxo-9 trifluorométhane-sulfonyloxy-7β taxène-11 yle-13α sous forme d'une meringue jaune pâle dont les caractéristiques sont les suivantes :
- spectre de R.M.N. ¹H (300 MHz ; CDCl₃ ; δ en ppm ; constantes de couplage J en Hz) : 0,38 (mt, 6H : CH₂ du triéthylsilyl en 2') ; 0,79 (t, J = 7,5, 9 H : CH₃ du triéthylsilyl en 2') ; 1,14 (s, 3H : CH₃) ; 1,28 (s, 3H : CH₃) ; 1,38 (s, 9H : C(CH₃)₃) ; 1,74 (s, 1H : OH en 1) ; 1,94 (s, 3H : CH₃) ; 1,98 (s, 3H : CH₃) ; 2,20 et 2,37 (2 dd, J = 16 et 9, 1H chacun : CH₂ en 14) ; de 2,25 à 2,40 et 2,84 (2 mt, 1H chacun : CH₂ en 6) ; 2,55 (s, 3H : COCH₃) ; 4,02 (s large, 1H : OH en 10) ; 4,04 (d, J = 7 Hz, 1H : H en 3) ; 4,24 et 4,38 (2d, J = 8,5, 1H chacun : CH₂ en 20) ; 4,54 (s large, 1H : H en 2') ; 4,96 (d large, J = 9,5, 1H : H en 5) ; 5,28 (d large, J = 10, 1H : H en 3') ; 5,38 (s large, 1H : H en 10) ; 5,44 (dd, J = 10 et 7,5, 1H : H en 7) ; 5,52 (d, J = 10, 1H : CONH) ; 5,74 (d, J = 7, 1H : H en 2) ; 6,34 (t large, J = 9, 1H: H en 13) ; de 7,25 à 7,40 (mt, 5H : H aromatiques en 3') ; 7,50 (t, J = 7,5, 2H : OCOC₆H₅ H en méta) ; 7,63 (t, J = 7,5, 1H : OCOC₆H₅ H en para) ; 8,12 (d, J = 7,5, 2H : OCOC₆H₅ H en ortho).

Le tert-butoxycarbonylamino-3 phényl-3 triéthylsilyloxy-2 propionate-(2R,3S) d'acétoxy-4α benzoyloxy-2α époxy-5β,20 trihydroxy-1β,7β,10β oxo-9 taxène-11 yle-13α peut être préparé de la manière suivante :

A une solution de 8,6 g de tert-butoxycarbonylamino-3 phényl-3 hydroxy-2 propionate-(2R,3S) d'acétoxy-4α benzoyloxy-2α époxy-5β,20 trihydroxy-1β,7β,10β oxo-9 taxène-11 yle-13α dans 40 cm3 de dichlorométhane anhydre et 8,6 cm3 de pyridine anhydre, à une température voisine de 20°C, sous atmosphère inerte d'argon, on ajoute goutte à goutte 8,05 cm3 de chlorure de triéthylsilyle. Le mélange réactionnel est agité, à une température voisine de 20°C, pendant 2 heures, puis on ajoute 300 cm3 de dichlorométhane. La phase organique est lavée par deux fois 50 cm3 d'eau distillée, 50 cm3 d'une solution aqueuse d'acide chlorhydrique 0,1N, 50 cm3 d'eau distillée, puis 50 cm3 d'une solution aqueuse saturée en chlorure de sodium, séchée sur sulfate de magnésium, filtrée sur verre fritté et concentrée sous pression réduite (2,7 kPa) à une température voisine de 40°C. On obtient ainsi 14,2 g d'une meringue blanche que l'on purifie par chromatographie à pression atmosphérique sur 800 g de silice (0,063-0,2 mm) contenus dans une colonne de 7 cm de diamètre en éluant avec un mélange méthanol-dichlorométhane (2-98 en volumes) en recueillant des fractions de 30 cm3. Les fractions ne contenant que le produit cherché sont réunies et concentrées à sec sous pression réduite (2,7 kPa) à 40°C pendant 2 heures. On obtient ainsi 8,85 g de tert-butoxycarbonylamino-3 phényl-3 triéthylsilyloxy-2 propionate-(2R,3S) d'acétoxy-4α benzoyloxy-2α époxy-5β,20 trihydroxy-1β,7β,10β oxo-9 taxène-11 yle-13α sous forme d'une meringue blanche.

### EXEMPLE 2

A une solution de 200 mg de tert-butoxycarbonylamino-3 phényl-3 triéthylsilyloxy-2 propionate-(2R,3S) d'acétoxy-4α benzoyloxy-2α époxy-5β,20 dihydroxy-1β,10β époxy-7α,9α taxène-11 yle-13α dans 2 cm3 de pyridine anhydre, maintenue sous atmosphère d'argon, à une température voisine de 20°C, on ajoute 0,0125 cm3 d'anhydride acétique, puis 13,5 mg de N,N'-diméthylamino-4-pyridine. Après 30 minutes à une température voisine de 20°C, le mélange réactionnel est dilué avec 40 cm3 d'acétate d'éthyle. La phase organique est lavée avec 2 fois 6 cm3 d'eau distillée, puis 6 cm3 d'une solution aqueuse saturée en chlorure de sodium, séchée sur sulfate de magnésium, filtrée sur verre fritté et concentrée sous pression réduite (2,7 kPa) à une température voisine de 40°C. On obtient ainsi 237,4 mg d'une meringue jaune pâle que l'on purifie par chromatographie à pression atmosphérique sur 20 g de silice (0,063-0,2 mm) contenus dans une colonne de 2,5 cm de diamètre en éluant avec un mélange acétate d'éthyle-dichlorométhane (gradient d'élution de 2-98 à 10-90 en volumes) en recueillant des fractions de 10 cm3. Les fractions ne contenant que le produit cherché sont réunies et concentrées à sec sous pression réduite (2,7 kPa) à 40°C pendant 2 heures. On obtient ainsi 184,8 mg de tert-butoxycarbonylamino-3 phényl-3 triéthylsilyloxy -2 propionate-(2R,3S) de diacétoxy-4α,10β benzoyloxy-2α époxy-5β,20 hydroxy-1β époxy-7α,9α taxène-11 yle-13α sous forme d'une meringue blanche dont les caractéristiques sont les suivantes :
- spectre de R.M.N. ¹H (400 MHz ; CDCl₃ ; δ en ppm ; constantes de couplage J en Hz) : 0,34 et 0,40 (2 mt, 6H : CH₂ du triéthylsilyl en 2') ; 0,76 (t, J = 7,5, 9 H : CH₃ du triéthylsilyl en 2') ; 1,26 (s, 3H : CH₃) ; 1,28 (s, 3H : CH₃) ; 1,38 (s, 9H : C(CH₃)₃) ; 1,72 (s, 3H : CH₃) ; 1,88 (s, 1H : OH en 1) ; 2,01 (s, 3H : CH₃) ; 2,14 (s, 3H : COCH₃) ; 2,23 et de 2,30 à 2,45 (respectivement dd et mt, J = 15 et 9, 1H chacun : CH₂ en 14) ; 2,39 (mt, 2H : CH₂ en 6) ; 2,48 (s, 3H : COCH₃) ; 4,05 et 4,30 (2 d, J = 7,5, 1H chacun : CH₂ en 20) ; 4,13 (d, J = 6, 1H : H en 3) ; 4,62 (s large, 1H : H en 2') ; 4,80 (t, J = 7,5, 1H : H en 7) ; 4,88 (d, J = 6, 1H : H en 9) ; 4,98 (s large, 1H : H en 5) ; 5,34 (d large, J = 10, 1H : H en 3') ; 5,54 (d, J = 10, 1H : CONH) ; 5,71 (d, J = 6, 1H : H en 10) ; 5,83 (d, J = 6, 1H : H en 2) ; 6,10 (t large, J = 9, 1H : H en 13) ; de 7,25 à 7,45 (mt, 5H : H aromatiques en 3') ; 7,48 (t, J = 7,5, 2H : OCOC₆H₅ H en méta) ; 7,62 (t, J = 7,5, 1H : OCOC₆H₅ H en para) ; 8,15 (d, J = 7,5, 2H : OCOC₆H₅ H en ortho).

A une solution de 180 mg de tert-butoxycarbonylamino-3 phényl-3 triéthylsilyloxy -2 propionate-(2R,3S) de diacétoxy-4α,10β benzoyloxy-2α époxy-5β,20 hydroxy-1β époxy-7α,9α taxène-11 yle-13α dans 1 cm3 de dichlorométhane anhydre, maintenue sous atmosphère d'argon, à une température voisine de 20°C, on ajoute goutte à goutte 0,93 cm3 de complexe acide fluorhydrique-triéthylamine (3HF.Et₃N). Après 7,5 heures à une température voisine de 20°C, le mélange réactionnel est dilué avec 30 cm3 d'acétate d'éthyle et 8 cm3 d'une solution aqueuse saturée en hydrogénocarbonate de sodium. Après décantation, la phase organique est lavée avec 2 fois 8 cm3 d'eau distillée, puis 8 cm3 d'une solution aqueuse saturée en chlorure de sodium, séchée sur sulfate de magnésium, filtrée sur verre fritté et concentrée sous pression réduite (2,7 kPa) à une température voisine de 40°C. On obtient ainsi 167,5 mg d'une meringue blanche que l'on purifie par chromatographie préparative sur couche mince de silice [9 plaques préparatives Merck, Kieselgel 60F254 ; 20 x 20 cm ; épaisseur 0,5 mm ; dépôt en solution dans le dichlorométhane], en éluant par un mélange méthanol-dichlorométhane (4-96 en volumes). Après élution de la zone correspondant au produit recherché par un mélange méthanol-dichiorométhane (15-85 en volumes), filtration sur verre fritté, puis évaporation des solvants sous pression réduite (2,7 kPa) à une température voisine de 40°C, on obtient 143,6 mg de tert-butoxycarbonylamino-3 phényl-3 hydroxy-2 propionate-(2R,3S) de diacétoxy-4α,10β benzoyloxy-2α époxy-5β,20 hydroxy-1β époxy-7α,9α taxène-11 yle-13α sous forme de meringue blanche dont les caractéristiques sont les suivantes :
- spectre de R.M.N. ¹H (400 MHz ; CDCl₃ ; δ en ppm ; constantes de couplage J en Hz) : 1,24 (s, 3H : CH₃) ; 1,32 (s, 3H : CH₃) ; 1,41 (s, 9H : C(CH₃)₃) ; 1,68 (s, 3H : CH₃) ; 1,91 (s, 1H : OH en 1) ; 1,92 (s, 3H : CH₃) ; 2,12 (s, 3H : COCH₃) ; 2,21 et de 2,25 à 2,55 (respectivement dd et mt, J = 15 et 8, 1H chacun : CH₂ en 14) ; de 2,25 à 2,55 (mt, 2H : CH₂ 6) ; 2,31 (s, 3H : COCH₃) ; 3,43 (mf, 1H : OH en 2') ; 4,03 et 4,30 (2 d, J = 8, 1H chacun : CH₂ en 20) ; 4,13 (d, J = 6, 1H : H en 3) ; 4,65 (mt, 1H : H en 2') ; 4,82 (dd, J = 8,5 et 5,5, 1H : H en 7) ; 4,86 (d, J = 6. 1H : H en 9) ; 4,93 (s large, 1H : H en 5) ; 5,34 (d large, J = 10, 1H : H en 3') ; 5,54 (d, J = 10, 1H : CONH) ; 5,65 (d, J = 6, 1H : H en 10) ; 5,83 (d, J = 6, 1H : H en 2) ; 6,03 (t large, J = 8, 1H : H en 13) ; 7,30 (t, J = 7,5, 1H : H en para de l'aromatique en 3') ; 7,38 (t, J = 7,5, 2H : H en méta de l'aromatique en 3') ; 7,43 (d, J = 7,5, 2H : H en ortho de l'aromatique en 3') ; 7,50 (t, J = 7,5, 2H : OCOC₆H₅ H en méta) ; 7,62 (t, J = 7,5, 1H : OCOC₆H₅ H en para) ; 8,13 (d, J = 7,5, 2H : OCOC₆H₅ H en ortho).

### EXEMPLE 3

A une solution de 149 mg de tert-butoxycarbonylamino-3 phényl-3 triéthylsilyloxy-2 propionate-(2R,3S) d'acétoxy-4α benzoyloxy-2α époxy-5β,20 hydroxy-1β époxy-7α,10α oxo-9 taxène-11 yle-13α, obtenu à l'exemple 1, dans 1,5 cm3 de dichlorométhane anhydre, maintenue sous atmosphère d'argon, à une température voisine de 20°C, on ajoute goutte à goutte 0,805 cm3 de complexe acide fluorhydrique-triéthylamine (3HF.Et₃N). Après 1 heure à une température voisine de 20°C, le mélange réactionnel est dilué avec 50 cm3 de dichlorométhane, 5 cm3 d'une solution aqueuse saturée en hydrogénocarbonate de sodium et 5 cm3 d'eau distillée. Après décantation, la phase organique est lavée avec 3 fois 8 cm3 d'eau distillée, puis 8 cm3 d'une solution aqueuse saturée en chlorure de sodium, séchée sur sulfate de magnésium, filtrée sur verre fritté et concentrée sous pression réduite (2,7 kPa) à une température voisine de 40°C. On obtient ainsi 133,2 mg d'une meringue jaune pâle que l'on purifie par chromatographie préparative sur couche mince de silice [10 plaques préparatives Merck, Kieselgel 60F254 ; 20 x 20 cm ; épaisseur 0,5 mm ; dépôt en solution dans le dichlorométhane], en éluant par un mélange méthanol-dichlorométhane (5-95 en volumes). Après élution de la zone correspondant au produit recherché par un mélange méthanol-dichlorométhane (15-85 en volumes), filtration sur verre fritté, puis évaporation des solvants sous pression réduite (2,7 kPa) à une température voisine de 40°C, on obtient 114,2 mg de tert-butoxycarbonylamino-3 phényl-3 hydroxy-2 propionate-(2R,3S) d'acétoxy-4α benzoyloxy-2α époxy-5β,20 hydroxy-1β époxy-7α,10α oxo-9 taxène-11 yle-13α sous forme de meringue blanche qui est purifié par chromatographie préparative sur couche mince de silice[8 plaques préparatives Merck, Kieselgel 60F254 ; 20 x 20 cm ; épaisseur 0,5 mm ; dépôt en solution dans le dichlorométhane], en éluant par un mélange méthanol-dichlorométhane (2-98 en volumes). Après élution de la zone correspondant au produit recherché par un mélange méthanol-dichlorométhane (15-85 en volumes), filtration sur verre fritté, puis évaporation des solvants sous pression réduite (2,7 kPa) à une température voisine de 40°C, on obtient 92,8 mg de tert-butoxycarbonylamino-3 phényl-3 hydroxy-2 propionate-(2R,3S) d'acétoxy-4α benzoyloxy-2α époxy-5β,20 hydroxy-1β époxy-7α,10α oxo-9 taxène-11 yle-13α sous forme de meringue blanche dont les caractéristiques sont les suivantes :
- spectre de R.M.N. ¹H (400 MHz ; CDCl₃ ; à une température de 333°K, δ en ppm ; constantes de couplage J en Hz) : 1,23 (s, 3H : CH₃) ; 1,33 (s, 3H : CH₃) ; 1,41 (s, 9H : C(CH₃)₃) ; 1,78 (s, 3H : CH₃) ; 1,83 (s, 3H : CH₃) ; 1,88 (s, 1H : OH en 1) ; 2,12 et 2,35 (2 dd, J = 15 et 8, 1H chacun : CH₂ en 14) ; 2,28 (s, 3H : COCH₃) ; 2,33 et 2,43 (2 dd, H en 7) ; 3,85 (mf, 1H : OH en 2') ; 4,28 (AB limite, J = 8, 2H : CH₂ en 20) ; 4,52 (d, J = 6,5, 1H : H en 3) ; 4,63 (mt, 1H : H en 2') ; 4,83 (mt, 1H : H en 10) ; 5,06 (d, J = 5, 1H : H en 5) ; 5,30 (d large, J = 10, 1H : H en 3') ; 5,53 (d, J = 10, 1H : CONH) ; 5,59 (d, J = 6,5, 1H : H en 2) ; 6,22 (t large, J = 8, 1H : H en 13) ; 7,30 (t, J = 7,5, 1H : H en para de l'aromatique en 3') ; 7,37 (t, J = 7,5, 2H : H en méta de l'aromatique en 3') ; 7,44 (d. J = 7,5, 2H : H en ortho de l'aromatique en 3') ; 7,50 (t, J = 7,5, 2H : OCOC₆H₅ H en méta) ; 7,61 (t, J = 7,5, 1H : OCOC₆H₅ H en para) ; 8,09 (d, J = 7,5, 2H : OCOC₆H₅ H en ortho).

### EXEMPLE 4

A une solution de 10 mg de tert-butoxycarbonylamino-3 phényl-3 triéthylsilyloxy-2 propionate-(2R,3S) d'acétoxy-4α benzoyloxy-2α époxy-5β,20 dihydroxy-1β,10β époxy-7α,9α taxène-11 yle-13α dans 0,1 cm3 d'iodure de méthyle et 0,01 cm3 de diméthylformamide anhydre, sous atmosphère d'argon, à une température voisine de 20°C, on ajoute 1 mg d'hydrure de sodium à 50 % dans l'huile. Après 12 minutes, à une température voisine de 20°C, le mélange réactionnel brut est purifié par chromatographie préparative sur couche mince de silice [1 plaque préparative Merck, Kieselgel 60F254 ; 20x20 cm ; épaisseur 0,5 mm ; dépôt du mélange réactionnel brut], en éluant par un mélange méthanol-dichlorométhane (3-97 en volumes). Après élution de la zone correspondant au produit recherché par un mélange méthanol-dichlorométhane (15-85 en volumes), filtration sur coton, puis évaporation des solvants sous pression réduite (2,7 kPa) à une température voisine de 40°C, on obtient 4,7 mg de tert-butoxycarbonylamino-3 phényl-3 triéthylsilyloxy-2 propionate-(2R,3S) d'acétoxy-4α benzoyloxy-2α époxy-5β,20 hydroxy-1β méthoxy-10β époxy-7α,9α taxène-11 yle-13a sous forme d'une laque blanche.

A une solution de 4 mg de tert-butoxycarbonylamino-3 phényl-3 triéthylsilyloxy-2 propionate-(2R,3S) d'acétoxy-4α benzoyloxy-2α époxy-5β,20 hydroxy-1β méthoxy-10β époxy-7α,9α taxène-11 yle-13α dans 0,1 cm3 de dichlorométhane anhydre, maintenue sous atmosphère d'argon, à une température voisine de 20°C, on ajoute goutte à goutte 0,01 cm3 de complexe acide fluorhydrique-triéthylamine (3HF.Et₃N). Après 35 minutes à une température voisine de 20°C, le mélange réactionnel brut est purifié par chromatographie préparative sur couche mince de silice [1 plaque préparative Merck, Kieselgel 60F254 ; 20 x 20 cm ; épaisseur 0,5 mm ; dépôt du mélange réactionnel brut], en éluant par un mélange méthanol-dichlorométhane (4-96 en volumes). Après élution de la zone correspondant au produit recherché par un mélange méthanol-dichlorométhane (15-85 en volumes), filtration sur coton, puis évaporation des solvants sous pression réduite (2,7 kPa) à une température voisine de 40°C, on obtient 3,3 mg de tert-butoxycarbonylamino-3 hydroxy-2 phényl-3 propionate-(2R,3S) d'acétoxy-4α benzoyloxy-2α époxy-5β,20 hydroxy-1β méthoxy-10β époxy-7α,9α taxène-11 yle-13α sous forme d'une laque blanche dont les caractéristiques sont les suivantes :
- spectre de R.M.N. ¹H (400 MHz ; CDCl₃ ; à une température de 333°K, δ en ppm ; constantes de couplage J en Hz) : 1,17 (s, 3H : CH₃) ; 1,22 (s, 3H : CH₃) ; 1,41 (s, 9H : C(CH₃)₃) ; 1,67 (s, 3H : CH₃) ; 1,94 (s, 1H : OH en 1) ; 2,00 (s, 3H : CH₃) ; 2,23 et 2,41 (2 dd, respectivement J = 15 et 8 et J = 15 et10 , 1H chacun : CH₂ en 14) ; de 2,20 à 2,40 (mt, 2H : CH₂ en 6) ; 2,31 (s, 3H : COCH₃) ; 3,33 (s, 3H : OCH₃) ; 4,03 (mf, 1H : OH en 2') ; 4,03 et 4,31 (2 d, J = 7,5, 1H chacun : CH₂ en 20) : 4,13 (d. J = 6,5, 1H : H en 3) ; 4,29 (d, J = 7, 1H : H en 9) ; 4,67 (mt, 1H : H en 2') ; 4,77 (dd, J = 8,5 et 5,5, 1H : H en 7) ; 4,90 (d, J = 7, 1H : H en 10) ; 4,93 (s large, 1H : H 5) ; 5,37 (d large, J = 10, 1H : H en 3') ; 5,61 (d, J = 10, 1H : CONH) ; 5,81 (d, J = 6,5, 1H : H en 2) ; 6,06 (mt, 1H : H en 13) ; 7,30 (t, J = 7,5, 1H : H en para de l'aromatique en 3') ; 7,38 (t, J = 7,5, 2H : H en méta de l'aromatique en 3') ; 7,46 (d, J = 7,5, 2H : H en ortho de l'aromatique en 3') ; 7,49 (t, J = 7,5, 2H : OCOC₆H₅ H en méta) ; 7,63 (t, J = 7,5, 1H : OCOC₆H₅ H en para) ; 8,13 (d, J = 7,5, 2H : OCOC₆H₅ H en ortho).

### EXEMPLE 5

Le tert-butoxycarbonylamino-3 phényl-3 hydroxy-2 propionate-(2R,3S) d'acétoxy-4a benzoyloxy-2α époxy-5β,20 hydroxy-1β oxa-7α,9α propanoyloxy-10β taxène-11 yle-13α peut être préparé de la manière suivante :

A une solution de 50 mg de tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β oxa-7α,9α propanoyloxy-10β taxène-11 yle-13α dans 0,5 cm3 d'acétate d'éthyle, maintenue à une température voisine de 20°C, on ajoute 0,0053 cm3 d'acide chlorhydrique concentré (36 %, d=1,18) Après 2 heures à une température voisine de 20°C, le mélange réactionnel brut est purifié par chromatographie préparative sur couche mince : 1 plaque préparative Merck, Kieselgel 60F254, 20x20 cm, épaisseur 1 mm, en éluant par un mélange méthanol-dichlorométhane (5-95 en volumes). Après élution de la zone correspondant au produit recherché par un mélange méthanol-dichlorométhane (15-85 en volumes), filtration sur verre fritté, puis évaporation des solvants sous pression réduite (2,7 kPa) à une température voisine de 40°C, on obtient 21 mg de tert-butoxycarbonylamino-3 phényl-3 hydroxy-2 propionate-(2R,3S) d'acétoxy-4α benzoyloxy-2α époxy-5β,20 hydroxy-1β oxa-7α,9α propanoyloxy-10β taxène-11 yle-13α sous forme d'une meringue blanche dont les caractéristiques sont les suivantes :
Spectre de R.M.N. ¹ H (400 MHz, CDCl₃, δ en ppm) : 1,18 (t, J = 7,5 Hz, 3H : CH₃ de l'éthyle) ; 1,26 (s, 3H : CH₃) ; 1,33 (s, 3H : CH₃) ; 1,41 (s, 9H : C(CH₃)₃) ; 1,69 (s, 3H : CH₃) ; 1,92 (s, 3H : CH₃) ; 2,23 et de 2,25 à 2,50 (respectivement dd et mt, J = 16 et 8 Hz, 1H chacun : CH₂ 14) ; de 2,25 à 2,50 (mt, 4H : CH₂ 6 et OCOCH₂ éthyle) ; 2,33 (s, 3H : COCH₃) ; 3,97 (s large, 1H : OH en 2') ; 4,03 et 4,31(2 d, J = 8 Hz, 1H chacun: CH₂ 20) ; 4,13 (d, J = 6 Hz, 1H : H 3) ; 4,68 (mt, 1H : H 2') ; 4,84 (dd. J = 8.5 et 5,5 Hz, 1H: H 7) ; 4,88 (d, J = 6 Hz, 1H: H 9) ; 4,96 (s large, 1H: H 5) ; 5,35 (d large, J = 10 Hz, 1H : H 3') ; 5,58 (d, J = 10 Hz, 1H : CONH) ; 5,69 et 5.85 (2 d, J = 6 Hz, 1H chacun : H 2 et H 10) ; 6,05 (t large, J = 8 Hz, 1H : H 13) ; 7,31 (t, J = 7,5 Hz, 1H : H en para de l'aromatique en 3') ; 7,39 (t, J = 7,5 Hz, 2H : H en méta de l'aromatique en 3') ; 7,46 (d, J = 7,5 Hz, 2H : H en ortho de l'aromatique en 3') ; 7,50 (t, J = 7,5 Hz, 2H : OCOC₆H₅ H méta) ; 7,53 (t, J = 7,5 Hz, 1H : OCOC₆H₅ H para) ; 8,13 (d, J = 7,5 Hz, 2H : OCOC₆H₅ H ortho).

Le tert-butoxycarbonylamino-3 (méthoxy-4 phényl)-2 phényl-4 oxazolidine-1,3 carboxylate-5 (2R,4S,5R) d'acétoxy-4a benzoyloxy-2α époxy-5β,20 hydroxy-1β oxa-7α,9α propanoyloxy-10β taxène-11 yle-13α peut être préparé de la manière suivante :

A une solution de 100 mg de tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 dihydroxy-1β,10β oxa-7α,9α taxène-11 yle-13α dans 1 cm3 de pyridine anhydre, maintenue sous atmosphère d'argon, à une température voisine de 20°C, on ajoute successivement 20 mg de N,N'-diméthylamino-4 pyridine puis 0,042 cm3 d'anhydride propionique. Après 2 heures à une température voisine de 20°C, le mélange réactionnel est dilué avec 5 cm3 de dichlorométhane et 2 cm3 d'eau distillée. Après décantation, la phase organique est séchée sur sulfate de magnésium, filtrée sur verre fritté et concentrée sous pression réduite (2,7 kPa) à une température voisine de 40°C. On obtient ainsi une huile incolore que l'on purifie par chromatographie préparative sur couche mince : 3 plaques préparatives Merck, Kieselgel 60F254, 20x20 cm, épaisseur 1 mm, dépôt en solution dans un volume minimum de dichlorométhane, en éluant par un mélange méthanol-dichlorométhane (5-95 en volumes). Après élution de la zone correspondant au produit recherché par un mélange méthanol-dichlorométhane (15-85 en volumes), filtration sur verre fritté, puis évaporation des solvants sous pression réduite (2,7 kPa) à une température voisine de 40°C, on obtient 51 mg de tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β oxa-7α,9α propanoyloxy-10β taxène-11 yle-13α sous forme de meringue blanche dont les caractéristiques sont les suivantes :
Spectre de R.M.N. ¹ H (400 MHz, CDCl₃, δ en ppm) : 1.04 (t, J = 7,5 Hz, 3H : CH₃ de l'éthyle) ; 1,05 (s, 9H : C(CH₃)₃) ; 1,24 (s, 6H : CH₃) ; 1,63 (s, 3H : CH₃) ; 1,70 (s, 3H : CH₃) ; 1,80 (s, 3H : COCH₃) ; 2,10 et de 2,15 à 2,55 (respectivement dd et mt, J = 16 et 8 Hz, 1H chacun : CH₂ 14) ; de 2,15 à 2,55 (mt, 4H : CH₂ 6 et OCOCH₂ éthyle) ; 3,80 (s, 3H : ArOCH₃) ; 3,92 et 4,22 (2 d, J = 8 Hz, 1H chacun : CH₂ 20) ; 4,02 (d, J = 6 Hz, 1H : H 3) ; 4,62 (d, J = 5 Hz, 1H : H 2') ; 4,73 (dd, J = 8 et 7,5 Hz, 1H : H 7) ; 4,78 (d, J = 6 Hz, 1H : H 9) ; 4,88 (s large, 1H : H 5) ; 5,35 (d large, J = 5 Hz, 1H : H 3') ; 5,63 et 5,75 (2 d, J = 6 Hz, 1H chacun : H 2 et H 10) ; 5,93 (t large, J = 8 Hz, 1H : H 13) ; 6,30 (s large, 1H : H 5') ; 6,89 (d, J = 8,5 Hz, 2H : H aromatiques en ortho du OCH₃) ; de 7,25 à 7,50 (mt, 9H : H aromatiques en 3' - H aromatiques en méta du OCH₃ et OCOC₆H₅ H méta) ; 7,58 (t, J = 7,5 Hz, 1H : OCOC₆H₅ H para) ; 8,03 (d, J = 7,5 Hz, 2H : OCOC₆H₅ H ortho).

Le tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 dihydroxy-1β,10β oxa-7α,9α taxène-11 yle-13α peut être préparé de la manière suivante :

A une solution de 1,1 g de tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β dioxo-9,10 trifluorométhanesulfonate-7β taxène-11 yle-13α dans 30 cm3 d'éthanol absolu, maintenue sous atmosphère d'argon, à une température voisine de 0°C, on ajoute 60 mg de borohydrure de sodium. Après une heure à une température voisine de 0°C, le mélange réactionnel est dilué avec 100 cm3 d'acétate d'éthyle. La phase organique est lavée avec 50 cm3 d'eau distillée, puis 2 fois 25 cm3 d'une solution aqueuse saturée en chlorure de sodium, séchée sur sulfate de magnésium, filtrée sur verre fritté et concentrée sous pression réduite (2,7 kPa) à une température voisine de 40°C. On obtient ainsi 1,04 g d'une meringue jaune pâle que l'on purifie par chromatographie à pression atmosphérique sur 50 g de silice (0,063-0,2 mm) contenus dans une colonne de 2,5 cm de diamètre en éluant avec un mélange méthanol-dichlorométhane (2-98 en volumes) en recueillant des fractions de 20 cm3. Les fractions ne contenant que le produit cherché sont réunies et concentrées à sec sous pression réduite (2,7 kPa) à 40°C pendant 2 heures. On obtient ainsi 230 mg de tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 dihydroxy-1β,10β oxa-7α,9α taxène-11 yle-13α sous forme d'une meringue blanche dont les caractéristiques sont les suivantes :
Spectre de R.M.N. ¹ H (400 MHz, CDCl₃, δ en ppm) : 1,05 (s, 9H : C(CH₃)₃) ; 1,10 (s, 3H : CH₃) ; 1,25 (s, 3H : CH₃) ; 1,58 (s, 3H : CH₃) ; 1,70 (s, 3H : CH₃) ; 1,85 (s large, 3H : COCH₃) ; 2,10 et 2,22 (2 dd, J = 16 et 8 Hz, 1H chacun : CH₂ 14) ; de 2,25 à 2,45 (mt, 2H : CH₂ 6) ; 3,82 (s, 3H : ArOCH₃) ; 3,93 et 4,23 (2 d, J = 8 Hz, 1H chacun : CH₂ 20) ; 4,08 (d, J = 6 Hz, 1H : H 3) ; 4,62 (d, J = 5 Hz, 1H : H 2') ; de 4,70 à 4,80 (mt, 2H : H 9 et H 10) ; 4,80 (dd, J = 8,5 et 6 Hz, 1H : H 7) ; 4,88 (s large, 1H : H 5) ; 5,36 (mf, 1H : H 3') : 5,75 (d, J = 6 Hz, 1H : H 2) ; 6,02 (t large, J = 8 Hz, 1H : H 13) ; 6,37 (mf étalé, 1H : H 5') ; 6,95 (d, J = 8,5 Hz, 2H : H aromatiques en ortho du OCH₃) ; de 7,25 à 7,55 (mt, 9H : H aromatiques en 3' - H aromatiques en méta du OCH₃ et OCOC₆H₅ H méta) ; 7,65 (t, J = 7,5 Hz, 1H : OCO C₆H₅ H para) ; 8,07 (d, J = 7,5 Hz, 2H : OCOC₆H₅ H ortho).

Le tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β dioxo-9,10 trifluorométhanesulfonate-7β taxène-11 yle-13α peut être préparé de la manière suivante :

A une suspension de 2,2 g de tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 dihydroxy-1β,10β oxo-9 trifluorométhanesulfonate-7β taxène-11 yle-13α et de 4,5 g de tamis moléculaire 4 Å activé dans 10 cm3 de dichlorométhane anhydre, maintenue sous atmosphère d'argon, à une température voisine de 20°C, on ajoute rapidement 1,8 g de chlorochromate de pyridinium. Le mélange réactionnel est agité pendant 17 heures, à une température voisine de 20°C, puis filtré sur Clarcel. Le résidu solide est rincé au dichlorométhane, puis le filtrat est concentré sous pression réduite (2,7 kPa) à une température voisine de 40°C. On obtient ainsi une meringue brune que l'on purifie par chromatographie à pression atmosphérique sur 200 g de silice (0,063-0,2 mm) contenus dans une colonne de 4 cm de diamètre en éluant avec un mélange méthanol-dichlorométhane (0,5-99,5 en volumes) en recueillant des fractions de 20 cm³. Les fractions ne contenant que le produit cherché sont réunies et concentrées à sec sous pression réduite (2,7 kPa) à 40°C pendant 2 h. On obtient ainsi 1,5 g de tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β dioxo-9,10 trifluorométhanesulfonate-7β taxène-11 yle-13α sous forme d'une meringue jaune dont les caractéristiques sont les suivantes :
Spectre de R.M.N. ¹ H (400 MHz, CDCl₃, δ en ppm) : 1,07 (s, 9H : C(CH₃)₃) ; 1,20 (s, 3H : CH₃) ; 1,27 (s, 3H : CH₃) ; 1,58 (s, 3H : CH₃) ; 1,85 (s, 3H : CH₃) ; 1,94 (mf, 3H : COCH₃) ; 2,13 et 2,27 (2 dd, J = 16 et 8 Hz, 1H chacun : CH₂ 14) ; 2,13 et 2,82 (2 mts, 1H chacun : CH₂ 6) ; 366 (d, J = 6,5 Hz, 1H : H 3) ; 3,84 (s, 3H : ArOCH₃) ; 4,11 et 4,31 (2 d, J = 8 Hz, 1H chacun : CH₂ 20) ; 4,58 (d, J = 5 Hz, 1H : H 2') ; 4,81 (d large, J = 10 Hz, 1H : H 5) ; 5,18 (dd, J = 10 et 7,5 Hz, 1H : H 7) ; 5,44 (mf, 1H : H 3') ; 5,77 (d, J = 6,5 Hz, 1H : H 2) ; 6,11 (t large, J = 8 Hz, 1H : H 13) ; 6,40 (mf, 1H : H 5') ; 6,91 (d, J = 8,5 Hz, 2H : H aromatiques en ortho du OCH₃) ; de 7,30 à 7,50 (mt, 7H : H aromatiques en 3' - H aromatiques en méta du OCH₃) ; 7,51 (t, J = 7,5 Hz, 2H : OCOC₆H₅ H méta) ; 7,66 (t, J = 7,5 Hz, 1H : OCO C₆H₅ H para) ; 8,02 (d, J = 7,5 Hz, 2H : OCOC₆H₅ H ortho).

La préparation du tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 dihydroxy-1β,10β oxo-9 trifluorométhanesulfonate-7β taxène-11 yle-13α peut être réalisée par exemple selon le mode opératoire décrit dans le brevet FR 9408198 (premier dépôt du 04/07/94).

### EXEMPLE 6

Le tert-butoxycarbonylamino-3 phényl-3 hydroxy-2 propionate-(2R,3S) d'acétoxy-4α benzoyloxy-2α époxy-5β,20 hydroxy-1β méthoxyacétoxy-10β oxa-7α,9α taxène-11 yle-13α peut être préparé de la manière suivante :

A une solution de 30 mg de tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β méthoxyacétoxy-10β oxa-7α,9α taxène-11 yle-13α dans 1 cm3 d'acétate d'éthyle, maintenue à une température voisine de 20°C, on ajoute 0,0053 cm3 d'acide chlorhydrique concentré (36 %, d=1,18). Après 45 minutes à une température voisine de 20°C, on ajoute 0,002 cm3 d'acide chlorhydrique concentré. Après 2 heures à une température voisine de 20°C, le mélange réactionnel brut est purifié par dépôt sur chromatographie préparative sur couche mince : 1 plaque préparative Merck, Kieselgel 60F254, 20x20 cm, épaisseur 0,5 mm, en éluant par un mélange méthanol-dichlorométhane (5-95 en volumes), Après élution de la zone correspondant au produit recherché par un mélange méthanol-dichlorométhane (15-85 en volumes), filtration sur verre fritté, puis évaporation des solvants sous pression réduite (2,7 kPa) à une température voisine de 40°C, on obtient 13 mg de tert-butoxycarbonylamino-3 phényl-3 hydroxy-2 propionate-(2R,3S) d'acétoxy-4α benzoyloxy-2α époxy-5β,20 hydroxy-1β méthoxyacétoxy-10β oxa-7α,9α taxène-11 yle-13α sous forme d'une meringue blanche dont les caractéristiques sont les suivantes :
Spectre de R.M.N. ¹ H (400 MHz, CDCl₃, δ en ppm) ; 1,24 (s, 3H ; CH3) ; 1,31 (s, 3H : CH₃) ; 1,40 (s, 9H : C(CH₃)₃) ; 1,67 (s, 3H : CH₃) ; 1,86 (s, 3H : CH₃) ; 1,99 (s, 1H : OH en 1) ; 2,20 et de 2,25 à 2,50 (respectivement dd et mt, J = 16 et 8 Hz, 1H chacun : CH₂ 14) ; de 2,25 à 2,50 (mt, 2H : CH₂ 6) ; 2,31 (s, 3H : COCH₃) ; 3,44 (s, 3H : OCH₃) ; 3,87 (s large, 1H : OH en 2') ; 4,01 et 4,18 (2 d, J = 8 Hz, 1H chacun : CH₂ 20) ; 4,04 et 4,11 (2 d, J = 16,5 Hz, 1H chacun : OCOCH₂O) ; 4,10 (d, J = 6 Hz, 1H : H 3) ; 4,64 (mt, 1H : H 2') ; 4,80 (dd, J = 8,5 et 5,5 Hz, 1H : H 7) ; 4,86 (d, J = 6 Hz, 1H : H 9) ; 4,92 (s large, 1H : H 5) ; 5,30 (d large, J = 10 Hz, 1H : H 3') ; 5,53 (d, J = 10 Hz, 1H : CONH) ; 5,74 et 5,80 (2 d, J = 6 Hz, 1H chacun : H 2 et H 10) ; 6,01 (t large, J = 8 Hz, 1H : H 13) ; 7,30 (t, J = 7,5 Hz, 1H : H en para de l'aromatique en 3') ; 7,36 (t, J = 7,5 Hz, 2H : H en méta de l'aromatique en 3') ; 7,42 (d, J = 7,5 Hz, 2H : H en ortho de l'aromatique en 3') ; 7,47 (t, J = 7,5 Hz, 2H : OCOC₆H₅ H méta) ; 7,61 (t, J = 7,5 Hz, 1H : OCOC₆H₅ H para) ; 8,11 (d, J = 7,5 Hz, 2H : OCOC₆H₅ H ortho).

Le tert-butoxycarbonylamino-3 (méthoxy-4 phényl)-2 phényl-4 oxazolidine-1.3 carboxylate-5 (2R,4S,5R) d'acétoxy-4α benzoyloxy-2α époxy-5β,20 hydroxy-1β méthoxyacétoxy-10β oxa-7α,9α taxène-11 yle-13α peut être préparé de la manière suivante :

A une solution de 90 mg de tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 dihydroxy-1β,10β oxa-7α,9α taxène-11 yle-13α dans 2 cm3 de tétrahydrofurane anhydre, maintenue sous atmosphère d'argon, à une température voisine de -78°C, on ajoute 0,093 cm3 de n.butyllithium (en solution 1,6M dans l'hexane), puis 8 minutes après 0,023 cm3 de chlorure de méthoxyacétyle. Le bain réfrigérant est retiré, puis le mélange réactionnel brut, après retour à une température voisine de 20°C, est purifie par dépôt sur chromatographie préparative sur couche mince : 2 plaques préparatives Merck, Kieselgel 60F254, 20x20 cm, épaisseur 1 mm, en éluant par un mélange méthanol-dichlorométhane (5-95 en volumes). Après élution de la zone correspondant au produit recherché par un mélange méthanol-dichlorométhane (15-85 en volumes), filtration sur verre fritté, puis évaporation des solvants sous pression réduite (2,7 kPa) à une température voisine de 40°C, on obtient 31 mg de tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β méthoxyacétoxy-10β oxa-7α,9α taxène-11 yle-13α sous forme de meringue blanche dont les caractéristiques sont les suivantes :
Spectre de R.M.N. ¹ H (400 MHz, CDCl₃, δ en ppm) : 1,05 (s, 9H : C(CH₃)₃) ; 1,26 (s, 6H : CH₃) ; 1,64 (s, 3H : CH₃) ; 1,67 (mf, 3H : CH₃) ; 1,78 (s, 3H : COCH₃) ; 2,10 et 2,21 (2 dd, J = 16 et 8,5 Hz, 1H chacun : CH₂ 14) ; 2,29 (mt, 2H : CH₂ 6) ; 3,45 (s, 3H : OCH₃) ; 3,81 (s, 3H : ArOCH₃) ; 3,92 et 4,24 (2 d, J = 8 Hz, 1H chacun : CH₂ 20) ; 4,00 (d, J = 6 Hz, 1H : H 3) ;4,03 et 4,10 (2 d, J = 16 Hz, 1H chacun : OCOCH₂O) ; 4,62 (d, J = 5 Hz, 1H : H 2') ; 4,75 (dd, J = 8 et 7,5 Hz, 1H: H 7) ; 4,82 (d, J = 6 Hz, 1H : H 9) ; 4,85(s large, 1H : H 5) ; 5,34 (mf, 1H : H 3') ; 5,68 et 5,75 (2 d, J = 6 Hz, 1H chacun : H 2 et H 10) ; 5,95 (mt, 1H : H 13) ; de 6,30 à 6,45 (mf très étalé, 1H : H 5') ; 6,92 (d, J = 8,5 Hz, 2H : H aromatiques en ortho du OCH₃) ; de 7,25 à 7,50 (mt, 9H : H aromatiques en 3' - H aromatiques en méta du OCH₃ et OCOC₆H₅ H méta) ; 7,62 (t, J = 7,5 Hz, 1H : OCOC₆H₅ H para) ; 8,06 (d, J = 7,5 Hz, 2H : OCOC₆H₅ H ortho).

### EXEMPLE 7

Le tert-butoxycarbonylamino-3 phényl-3 hydroxy-2 propionate-(2R,3S) d'acétoxy-4α benzoyloxy-2α époxy-5β,20 hydroxy-1β diméthylaminocarbonyloxy-10β oxa-7α,9α taxène-11 yle-13α peut être préparé de la manière suivante :

A une solution de 40 mg de tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β diméthylaminocarbonyloxy-10β oxa-7α,9α taxène-11 yle-13α dans 1 cm3 d'acétate d'éthyle, maintenue à une température voisine de 20°C, on ajoute 0,0043 cm3 d'acide chlorhydrique concentré (36 %, d=1,18). Après 1,5 heure à une température voisine de 20°C, le mélange réactionnel brut est purifié par dépôt sur chromatographie préparative sur couche mince : 2 plaques préparatives Merck, Kieselgel 60F254, 20x20 cm, épaisseur 0,5 mm, en éluant par un mélange méthanol-dichlorométhane (5-95 en volumes). Après élution de la zone correspondant au produit recherché par un mélange méthanol-dichlorométhane (15-85 en volumes), filtration sur verre fritté, puis évaporation des solvants sous pression réduite (2,7 kPa) à une température voisine de 40°C, on obtient 20 mg de tert-butoxycarbonylamino-3 phényl-3 hydroxy-2 propionate-(2R,3S) d'acétoxy-4α benzoyloxy-2α époxy-5β,20 hydroxy-1β diméthylaminocarbonyloxy-10β oxa-7α,9α taxène-11 yle-13α sous forme d'une meringue blanche dont les caractéristiques sont les suivantes :
Spectre de R.M.N. ¹ H (400 MHz, CDCl₃, δ en ppm) : 1,24 (s, 3H : CH₃) ; 1,31 (s, 3H : CH₃) ; 1,40 (s, 9H : C(CH₃)₃) ; 1,69 (s, 3H : CH₃) ; 1,91 (s, 1H : OH en 1) ; 1,97 (s, 3H : CH₃) ; 2,22 et de 2,25 à 2,50 (respectivement dd et mt, J = 16 et 8 Hz, 1H chacun : CH₂ 14) ; de 2,25 à 2,50 (mt, 2H : CH₂ 6) ; 2,32 (s, 3H : COCH₃) ; 2,94 et 2,96 (2 s, 3H chacun : N(CH₃)₂) ; 3,96 (s large, 1H : OH en 2') ; 4,03 et 4,31 (2 d, J = 8 Hz, 1H chacun : CH₂ 20) ; 4,13 (d, J = 6 Hz, 1H : :H3) 4,67 (mt, 1H : H 2') ; 4,81 (dd, J = 8,5 et 5,5 Hz. 1H : H 7) ; 4,91 (d, J = 6 Hz, 1H : H 9) ; 4,95 (s large, 1H : H 5) ; 5,24 (d large, J = 10 Hz, 1H : H 3') ; 5,58 (d, J = 10 Hz, 1H : CONH) ; 5,69 et 5,83 (2 d, J = 6 Hz, 1H chacun : H 2 et H 10) ; 6,07 (t large, J = 8 Hz, 1H : H 13) ; 7,31 (t, J = 7,5 Hz, 1H : H en para de l'aromatique en 3') ; 7,39 (t, J = 7,5 Hz, 2H : H en méta de l'aromatique en 3') ; 7,46 (d, J = 7,5 Hz, 2H : H en ortho de l'aromatique en 3') ; 7,48 (t, J = 7,5 Hz, 2H : OCOC₆H₅ H méta) ; 7,62 (t, J = 7,5 Hz, 1H : OCOC₆H₅ H para) ; 8,13 (d, J = 7,5 Hz, 2H : OCOC₆H₅ H ortho).

Le tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β diméthylaminocarbonyloxy-10β oxa-7α,9α taxène-11 yle-13α peut être préparé de la manière suivante :

A une solution de 100 mg de tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 dihydroxy-1β,10β oxa-7α,9α taxène-11 yle-13α dans 2 cm3 de tétrahydrofurane anhydre, maintenue sous atmosphère d'argon, à une température voisine de -78°C, on ajoute 0,103 cm3 de n-butyllithium (en solution 1,6M dans l'hexane), puis 5 minutes après 0,0253 cm3 de chlorure de diméthylaminocarbamoyle. Après 30 minutes à une température voisine de -78°C, le bain réfrigérant est retiré, puis le mélange réactionnel brut, après retour à une température voisine de 20°C, est dilué par 1 cm3 d'eau distillée. Après décantation, la phase aqueuse est réextraite par 2 cm3 d'acétate d'éthyle. Les phases organiques réunies sont séchées sur sulfate de magnésium, filtrées sur verre fritté et concentrées sous pression réduite (2,7 kPa) à une température voisine de 40°C. On obtient ainsi 99 mg d'une laque incolore que l'on purifie par chromatographie préparative sur couche mince : 2 plaques préparatives Merck, Kieselgel 60F254, 20x20 cm, épaisseur 1 mm, dépôt en solution dans un minimum de dichlorométhane, en éluant par un mélange méthanol-dichlorométhane (5-95 en volumes). Après élution de la zone correspondant au produit recherché par un mélange méthanol-dichlorométhane (15-85 en volumes), filtration sur verre fritté, puis évaporation des solvants sous pression réduite (2,7 kPa) à une température voisine de 40°C, on obtient 43 mg de tert-butoxycarbonylamino-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β diméthylaminocarbonyloxy-10β oxa-7α,9α taxène-11 yle-13α sous forme de meringue blanche dont les caractéristiques sont les suivantes :
Spectre de R.M.N. ¹ H (400 MHz, CDCl₃, δ en ppm) : 1,05 (s, 9H : C(CH₃)₃) ; 1,24 (s, 6H : CH₃) ; 1,61 (s, 3H : CH₃) ; 1,70 (s, 3H : CH₃) ; 1,81 (s, 1H : OH en 1) ; 1,87 (s, 3H : COCH₃) ; 2,14 et de 2,15 à 2,35 (respectivement dd et mt, J = 16 et 8 Hz, 1H chacun : CH₂ 14) ; de 2,15 à 2,35 (mt, 2H : CH₂ 6) ; 2.92 (s, 6H : N(CH₃)₂) ; 3,80 (s, 3H : ArOCH₃) ; 3,92 et 4,23 (2 d, J = 8 Hz, 1H chacun : CH₂ 20) ; 4,02 (d, J = 6 Hz, 1H : H 3) ; 4,62 (d, J = 5 Hz, 1H : H 2') ; 4,71 (dd, J = 8 et 7,5 Hz, 1H : H 7) ; 4,83 (d, J = 6 Hz, 1H : H 9) ; 4,88 (s large, 1H : H 5) ; 5,35 (d large, J = 5 Hz, 1H : H 3') ; 5,68 et 5,77 (2 d, J = 6 Hz, 1H : H 2 et H 10) ; 6,00 (t large, J = 8 Hz, 1H : H 13) ; 6,30 (s, 1H : H 5') ; 6,92 (d, J = 8,5 Hz, 2H : H aromatiques en ortho du OCH₃) ; de 7,25 à 7,50 (mt, 9H : H aromatiques en 3' - H aromatiques en méta du OCH₃ et OCOC₆H₅ H méta) ; 7,60 (t, J = 7,5 Hz, 1H : OCOC₆H₅ H para) ; 8,05 (d, J = 7,5 Hz, 2H : OCOC₆H₅ H ortho).

Les nouveaux produits de formule générale (I) dans laquelle Z représente un radical de formule générale (II) manifestent une activité inhibitrice significative de la prolifération cellulaire anormale et possèdent des propriétés thérapeutiques permettant le traitement de malades ayant des conditions pathologiques associées à une prolifération cellulaire anormale. Les conditions pathologiques incluent la prolifération cellulaire anormale de cellules malignes ou non malignes de divers tissus et/ou organes, comprenant, de manière non limitative, les tissus musculaires, osseux ou conjonctifs, la peau, le cerveau, les poumons, les organes sexuels, les systèmes lymphatiques ou rénaux, les cellules mammaires ou sanguines, le foie, l'appareil digestif, le pancréas et les glandes thyroïdes ou adrénales. Ces conditions pathologiques peuvent inclure également le psoriasis, les tumeurs solides, les cancers de l'ovaire, du sein, du cerveau, de la prostate, du colon, de l'estomac, du rein ou des testicules, le sarcome de Kaposi, le cholangiocarcinome, le choriocarcinome, le neuroblastome, la tumeur de Wilms, la maladie de Hodgkin, les mélanomes, les myélomes multiples, les leucémies lymphocytaires chroniques, les lymphomes granulocytaires aigus ou chroniques. Les nouveaux produits selon l'invention sont particulièrement utiles pour le traitement du cancer de l'ovaire. Les produits selon l'invention peuvent être utilisés pour prévenir ou retarder l'apparition ou la réapparition des conditions pathologiques ou pour traiter ces conditions pathologiques.

Les produits selon l'invention peuvent être administrés à un malade selon différentes formes adaptées à la voie d'administration choisie qui, de préférence, est la voie parentérale ou la voie orale. L'administration par voie parentérale comprend les administrations intraveineuse, intrapéritonéale, intramusculaire ou sous-cutanée. Plus particulièrement préférée est l'administration intrapéritonéale ou intraveineuse.

La présente invention comprend également les compositions pharmaceutiques qui contiennent au moins un produit de formule générale (I) en une quantité suffisante adaptée à l'emploi en thérapeutique humaine ou vétérinaire. Les compositions peuvent être préparées selon les méthodes habituelles en utilisant un ou plusieurs adjuvants, supports ou excipients pharmaceutiquement acceptables. Les supports convenables incluent les diluants, les milieux aqueux stériles et divers solvants non toxiques. De préférence les compositions se présentent sous forme de solutions ou de suspensions aqueuses, de solutions injectables qui peuvent contenir des agents émusifiants, des colorants, des préservatifs ou des stabilisants. Cependant, les compositions peuvent aussi se présenter sous forme de comprimés, de pilules, de poudres ou de granulés administrables par voie orale.

Le choix des adjuvants ou excipients peut être déterminé par la solubilité et les propriétés chimiques du produit, le mode particulier d'administration et les bonnes pratiques pharmaceutiques.

Pour l'administration parentérale, on utilise des solutions ou des suspensions stériles aqueuses ou non aqueuses. Pour la préparation de solutions ou de suspensions non aqueuses peuvent être utilisés des huiles végétales naturelles telle que l'huile d'olive, l'huile de sésame ou l'huile de paraffine ou les esters organiques injectables tel que l'oléate d'éthyle. Les solutions stériles aqueuses peuvent être constituées d'une solution d'un sel pharmaceutiquement acceptable en solution dans de l'eau. Les solutions aqueuses conviennent pour l'administration intraveineuse dans la mesure où le pH est convenablement ajusté et où l'isotonicité est réalisée, par exemple, par une quantité suffisante de chlorure de sodium ou de glucose. La stérilisation peut être réalisée par chauffage ou par tout autre moyen qui n'altère pas la composition.

Il est bien entendu que tous les produits entrant dans les compositions selon l'invention doivent être purs et non toxiques pour les quantités utilisées.

Les compositions peuvent contenir au moins 0,01 % de produit thérapeutiquement actif. La quantité de produit actif dans une composition est telle qu'une posologie convenable puisse être prescrite. De préférence, les compositions sont préparées de telle façon qu'une dose unitaire contienne de 0,01 à 1000 mg environ de produit actif pour l'administration par voie parentérale.

Le traitement thérapeutique peut être effectué concuremment avec d'autres traitements thérapeutiques incluant des médicaments antinéoplastiques , des anticorps monoclonaux, des thérapies immunologiques ou des radiothérapies ou des modificateurs des réponses biologiques. Les modificateurs des réponses incluent, de manière non limitative, les lymphokines et les cytokines telles que les interleukines, les interférons (α, β ou δ) et le TNF. D'autres agents chimiothérapeutiques utiles dans le traitement des désordres dus à la prolifération anormale des cellules incluent, de manière non limitative, les agents alkylants tels que les moutardes à l'azote comme la mechloretamine, le cyclophosphamide, le melphalan et le chlorambucil, des sulfonates d'alkyle comme le busulfan, les nitrosourées comme la carmustine, la lomustine, la sémustine et la streptozocine, les triazènes comme la dacarbazine, les antimétabolites comme les analogues de l'acide folique tel que le méthotrexate, les analogues de pyrimidine comme le fluorouracil et la cytarabine, des analogues de purines comme la mercaptopurine et la thioguanine, des produits naturels tels que les alcaloïdes de vinca comme la vinblastine, la vincristine et la vendésine, des épipodophyllotoxines comme l'étoposide et le teniposide, des antibiotiques comme la dactinomycine, la daunorubicine, la doxorubicine, la bléomycine, la plicamycine et la mitomycine, des enzymes comme la L-asparaginase, des agents divers comme les complexes de coordination du platine tel que le cisplatine, les urées substituées telles que l'hydroxyurée, les dérivés de méthylhydrazine comme la procarbazine, les suppresseurs adrénocoticoïdes comme le mitotane et l'aminoglutéthymide, les hormones et les antagonistes comme les adrénocorticostéroïdes comme la prednisone, les progestines comme le caproate d'hydroxyprogestérone, l'acétate de méthoxyprogestérone et l'acétate de megestrol, les oestrogènes comme le diéthylstilbestrol et l'éthynylestradiol, les antioestrogènes comme le tamoxifène, les androgènes comme le propionate de testostérone et la fluoxymesterone.

Les doses utilisées pour mettre en oeuvre les méthodes selon l'invention sont celles qui permettent un traitement prophylactique ou un maximum de réponse thérapeutique. Les doses varient selon la forme d'administration, le produit particulier sélectionné et les caractéristiques propres du sujet à traiter. En général, les doses sont celles qui sont thérapeutiquement efficaces pour le traitement des désordres dus à une prolifération cellulaire anormale. Les produits selon l'invention peuvent être administrés aussi souvent que nécessaire pour obtenir l'effet thérapeutique désiré. Certains malades peuvent répondre rapidement à des doses relativement fortes ou faibles puis avoir besoin de doses d'entretien faibles ou nulles. Généralement, de faibles doses seront utilisées au début du traitement et, si nécessaire, des doses de plus en plus fortes seront administrées jusqu'à l'obtention d'un effet optimum. Pour d'autres malades il peut être nécessaire d'administrer des doses d'entretien 1 à 8 fois par jour, de préférence 1 à 4 fois, selon les besoins physiologiques du malade considéré. Il est aussi possible que pour certains malades il soit nécessaire de n'utiliser qu'une à deux administrations journalières.

Chez l'homme, les doses sont généralement comprises entre 0,01 et 200 mg/kg. Par voie intrapéritonéale, les doses seront en général comprises entre 0,1 et 100 mg/kg et, de préférence entre 0,5 et 50 mg/kg et, encore plus spécifiquement entre 1 et 10 mg/kg. Par voie intraveineuse, les doses sont généralement comprises entre 0,1 et 50 mg/kg et, de préférence entre 0,1 et 5 mg/kg et, encore plus spécifquement entre 1 et 2 mg/kg. Il est entendu que, pour choisir le dosage le plus approprié, devront être pris en compte la voie d'administration, le poids du malade, son état de santé général, son âge et tous les facteurs qui peuvent influer sur l'efficacité du traitement.

L'exemple suivant illustre une composition selon l'invention.

### EXEMPLE

On dissout 40 mg du produit obtenu à l'exemple 1 dans 1 cm3 d'Emulphor EL 620 et 1 cm3 d'éthanol puis la solution est diluée par addition de 18 cm3 de sérum physiologique.

La composition est administrée par perfusion pendant 1 heure par introduction dans du soluté physiologique

## Revendications

1. Nouveaux taxoïdes de formule générale : 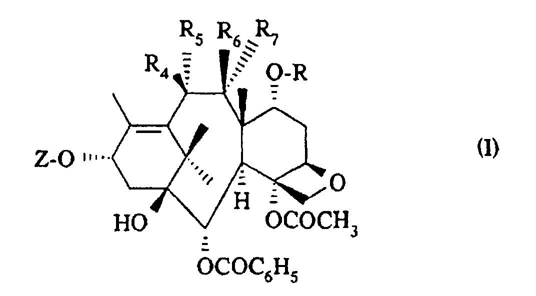 dans laquelle :
Z représente un atome d'hydrogène ou un radical de formule générale : 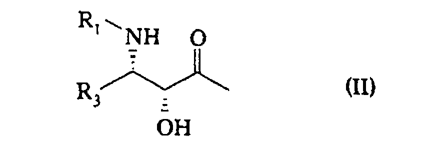
dans laquelle :
R₁ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle ou un radical R₂-O-CO- dans lequel R₂ représente un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoyiamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone, un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle, ou un radical hétérocyclyle saturé contenant 4 à 6 atomes de carbone éventuellement substitué par un ou plusieurs radicaux alcoyles contenant 1 à 4 atomes de carbone,
R₃ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, cycloalcényles contenant 4 à 6 atomes de crabone, phényle ou α- ou β-naphtyle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonyl-amino, amino, alcoylamino, dialcoylamino, carboxy, alcoxycarbonyle, carbamoyle, alcoylcarbamoyle, dialcoylcarbamoyle, cyano, nitro et trifluorométhyle, ou un hétérocycle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles,
ou bien R₄ représente un atome d'hydrogène,
R₆ et R₇ forment ensemble une fonction cétone, et
R et R₅ forment ensemble une liaison,
ou bien R₄ représente un atome d'hydrogène ou un radical hydroxy ou un radical alcoxy contenant 1 à 6 atomes de carbone en chaîne droite ou ramifiée, alcényloxy contenant 3 à 6 atomes de carbone en chaîne droite ou ramifiée, alcynyloxy contenant 3 à 6 atomes de carbone en chaîne droite ou ramifiée, cycloalcoyloxy contenant 3 à 6 atomes de carbone, cycloalcényloxy contenant 3 à 6 atomes de carbone, alcanoyloxy dont la partie alcanoyle contient 1 à 6 atomes de carbone en chaîne droite ou ramifiée, aroyloxy dont la partie aryle contient 6 à 10 atomes de carbone, alcénoyloxy dont la partie alcénoyle contient 3 à 6 atomes de carbone en chaîne droite ou ramifiée, alcynoyloxy dont la partie alcynoyle contient 3 à 6 atomes de carbone en chaîne droite ou ramifiée, cycloalcanoyloxy contenant 3 à 6 atomes de crabone, alcoxyacétyle dont la partie alcoyle contient 1 à 6 atomes de carbone en chaîne droite ou ramifiée, alcoylthioacétyle dont la partie alcoyle contient 1 à 6 atomes de carbone en chaîne droite ou ramifiée, alcoyloxycarbonyloxy dont la partie alcoyle contient 1 à 6 atomes de carbone en chaîne droite ou ramifiée, ces radicaux étant éventuellement substitués par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone, ou bien R₄ représente un radical carbamoyloxy, alcoylcarbamoyloxy dont la partie alcoyle contient 1 à 4 atomes de carbone, dialcoylcarbamoyloxy dont chaque partie alcoyle contient 1 à 4 atomes de carbone ou un radical benzoyloxy ou hétérocyclylcarbonyloxy dans lequel la partie hétérocyclique représente un hétérocycle aromatique à 5 ou 6 chaînons contenant un ou plusieurs hétéroatomes choisis parmi les atomes d'oxygène, de soufre ou d'azote,
R₅ représente un atome d'hydrogène,
ou R₄ et R₅ forment ensemble une fonction cétone,
R₆ représente un atome d'hydrogène, et
R et R₇ forment ensemble une liaison.

2. Nouveaux taxoïdes selon la revendication 1 pour lesquels Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical alcoyle contenant 1 à 6 atomes de carbone, alcényle contenant 2 à 6 atomes de carbone, cycloalcoyle contenant 3 à 6 atomes de carbone, phényle éventuellement substitué par ou plusieurs atomes ou radicaux, identiques ou différents, choisis parmi les les atomes et les radicaux alcoyles, alcoxy, dialcoylamino, acylamino, alcoxycarbonylamino ou trifluorométhyle ou un radical furyle-2 ou -3, thiényle-2 ou -3 ou thiazolyle-2, -4 ou -5 et, ou bien R₄ représente un atome d'hydrogène, R₆ et R₇ forment ensemble une fonction cétone et R et R₅ forment ensemble une liaison, ou bien R₄ représente un radical hydroxy, alcoxy contenant 1 à 6 atomes de carbone, alcanoyloxy contenant 1 à 6 atomes de carbone ou alcoxyacétyle dont la partie alcoyle contient 1 à 6 atomes de carbone, R₅ représente un atome d'hydrogène, R₆ représente un atome d'hydrogène et R et R₇ forment ensemble une liaison, ou bien R₄ et R₅ forment ensemble une fonction cétone, R₆ représente un atome d'hydrogène, R et R₇ forment ensemble forment ensemble une liaison.

3. Nouveaux taxoïdes selon la revendication 1 pour lesquels Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyle-2, furyle-3, thiényle-2, thiényle-3, thiazolyle-2, thiazolyle-4 ou thiazolyle-5, ou bien R₄ représente un atome d'hydrogène, R₆ et R₇ forment ensemble une fonction cétone et R et R₅ forment ensemble une liaison, ou bien R₄ représente un radical hydroxy ou un radical méthoxy, acétoxy, propanoyloxy ou méthoxyacétoxy, R₅ représente un atome d'hydrogène, R₆ représente un atome d'hydrogène et R et R₇ forment ensemble une liaison.

4. Procédé de préparation d'un produit selon l'une des revendications 1, 2 ou 3 dans laquelle, ou bien R₄ représente un atome d'hydrogène, R₆ et R₇ forment ensemble une fonction cétone et R et R₅ forment ensemble une liaison, ou bien R₄ représente un radical hydroxy, R₅ représente un atome d'hydrogène, R₆ représente un atome d'hydrogène et R et R₇ forment ensemble une liaison, **caractérisé en ce que** l'on fait réagir un agent de réduction sur un produit de formule générale : 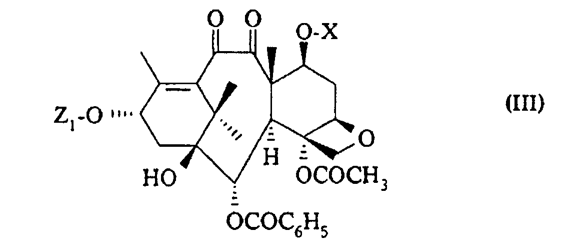 dans laquelle Z₁ représente un atome d'hydrogène ou un groupement protecteur de la fonction hydroxy ou un radical de formule générale : 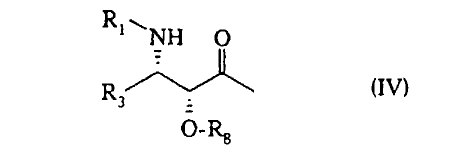 dans laquelle R₁ et R₃ sont définis comme dans l'une des revendications 1, 2 ou 3 et R₈ représente un groupement protecteur de la fonction hydroxy et X représente, avec l'atome d'oxygène auquel il est lié, un groupe partant choisi parmi les radicaux alcoylsulfonyle contenant 1 à 4 atomes de carbone éventuellement substitué par un ou plusieurs atomes d'halogène, arylsulfonyle dont la partie aryle est un radical phényle éventuellement substitué par un ou plusieurs atomes ou radicaux, identiques ou différents, choisis parmi les atomes d'halogène et les radicaux alcoyle contenant 1 à 4 atomes de carbone, nitro ou trifluorométhyle, pour obtenir un produit de formule générale : 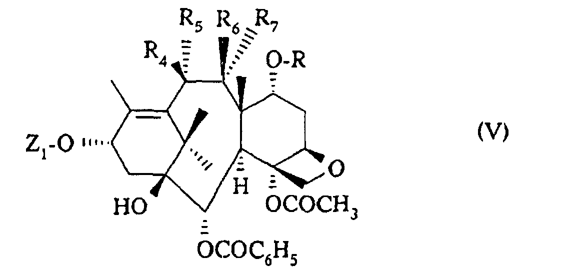 dans laquelle Z₁, R, R₄, R₅, R₆ et R₇ sont définis comme précédemment, sous forme un mélange d'un produit de formule générale (I) dans laquelle R₄ représente un atome d'hydrogène, R₆ et R₇ forment ensemble une fonction cétone, et R et R₅ forment ensemble une liaison, et d'un produit de formule générale (I) dans laquelle R₄ représente radical hydroxy, R₅ représente un atome d'hydrogène, R₆ représente un atome d'hydrogène, et R et R₇ forment ensemble une liaison que l'on sépare par les méthodes habituelles, puis on remplace éventuellement le groupement protecteur représenté par Z₁ ou R₈ par un atome d'hydrogène.

5. Procédé selon la revendication 4 **caractérisé en ce que** l'agent de réduction est choisi parmi les aluminohydrures et les hydroborures tels que hydroborures de métal alcalin, ou alcalino-terreux en présence d'un alcool aliphatique contenant 1 à 4 atomes de carbone tel que l'éthanol, la réaction étant mise en oeuvre à une température comprise entre 0 et 50°C.

6. Procédé selon la revendication 4 **caractérisé en ce que**, lorsque le groupement protecteur, représente un radical silylé, on le remplace par un atome d'hydrogène au moyen d'un acide minéral dans un alcool aliphatique contenant 1 à 3 atomes de carbone à une température comprise entre -10 et 20°C ou au moyen du complexe acide fluorhydrique-triéthylamine en opérant dans un solvant organique inerte à une température comprise entre 0 et 50°C.

7. Procédé de préparation d'un produit selon l'une des revendications 1, 2 ou 3 pour lequel Z est défini comme dans l'une des revendications 1, 2 ou 3, R₄ représente un radical alcoxy contenant 1 à 6 atomes de carbone en chaîne droite ou ramifiée, alcényloxy contenant 3 à 6 atomes de carbone en chaîne droite ou ramifiée, alcynyloxy contenant 3 à 6 atomes de carbone en chaîne droite ou ramifiée, cycloalcoyloxy contenant 3 à 6 atomes de carbone, cycloalcényloxy contenant 3 à 6 atomes de carbone, alcanoyloxy dont la partie alcanoyle contient 1 à 6 atomes de carbone en chaîne droite ou ramifiée, alcénoyloxy dont la partie alcénoyle contient 3 à 6 atomes de carbone en chaîne droite ou ramifiée, alcynoyloxy dont la partie alcynoyle contient 3 à 6 atomes de carbone en chaîne droite ou ramifiée, cycloalcanoyloxy contenant 1 à 6 atomes de carbone, alcoxyacétyle dont la partie alcoyle contient 1 à 6 atomes de carbone en chaîne droite ou ramifiée, alcoylthioacétyle dont la partie alcoyle contient 1 à 6 atomes de carbone en chaîne droite ou ramifiée, alcoyloxycarbonyloxy dont la partie alcoyle contient 1 à 6 atomes de carbone en chaîne droite ou ramifiée, ces radicaux étant éventuellement substitués par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone, ou bien R₄ représente un radical carbamoyloxy, alcoylcarbamoyloxy dont la partie alcoyle contient 1 à 4 atomes de carbone ou dialcoylcarbamoyloxy dont chaque partie alcoyle contient 1 à 4 atomes de carbone ou un radical benzoyloxy ou hétérocyclylcarbonyloxy dans lequel la partie hétérocyclique représente un hétérocycle aromatique à 5 ou 6 chaînons contenant un ou plusieurs hétéroatomes choisis parmi les atomes d'oxygène, de soufre ou d'azote, R₅ représente un atome d'hydrogène, R₆ représente un atome d'hydrogène, et R et R₇ forment ensemble une liaison, **caractérisé en ce que** l'on fait réagir un produit de formule générale :
R'₄-Y (IX)
dans laquelle R'₄ est tel que R'₄-O- est identique à R₄ défini comme précédemment et Y représente un groupe partant, sur un produit de formule générale : 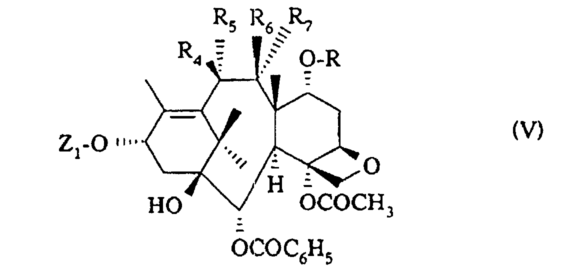 dans laquelle Z₁ est défini comme précédemment, R₄ représente un radical hydroxy, R6 représente un atome d'hydrogène et R et R₇ forment ensemble une liaison, suivi du remplacement du groupement protecteur représenté par Z₁ ou R₈ par un atome d'hydrogène dans les conditions de la revendication 6.

8. Procédé selon la revendication 7 **caractérisé en ce que**, préalablement à l'action du produit de formule générale (IX) on métalle éventuellement la fonction hydroxy en position 10 au moyen d'un hydrure, d'un amidure ou d'un alcoylure de métal alcalin.

9. Procédé de préparation d'un produit selon l'une des revendications 1, 2 ou 3 pour lequel Z représente un radical de formule générale (II), R₄ et R₅ forment ensemble une fonction cétone, R₆ représente un atome d'hydrogène, et R et R₇ forment ensemble une liaison, **caractérisé en ce que** l'on oxyde un produit de formule générale : 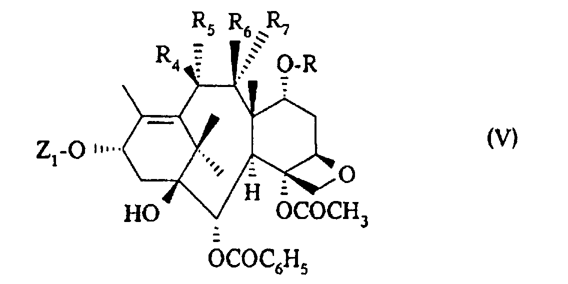 dans laquelle Z₁ est défini comme dans la revendication 4, R₄ représente un radical hydroxy, R₅ représente un atome d'hydrogène, R₆ représente un atome d'hydrogène et R et R₇ forment ensemble une liaison, puis on remplace éventuellement le groupement protecteur représenté par Z₁ ou R₈ par un atome d'hydrogène dans les conditions de la revendication 6.

10. Procédé selon la revendication 9 **caractérisé en ce que** l'oxydation est réalisée au moyen d'oxygène, de peruthénate d'ammonium, de bioxyde de manganèse, d'acétate de cuivre ou de chlorochromate de pyridinium.

11. Procédé de préparation d'un produit selon l'une des revendications 1, 2 ou 3 pour lequel Z représente un radical de formule générale (II), R₄, R₅ et R₆ représentent chacun un atome d'hydrogène et R et R₇ forment ensemble une liaison, peuvent être obtenus à partir d'un produit de formule générale (V) dans laquelle Z₁ est défini comme dans la revendication 4, R₄ représente un radical hydroxy, R₅ et R₆ représentent chacun un atome d'hydrogène et R et R₇ forment ensemble une liaison, après transformation du radical hydroxy représenté par R₄ en dithiocarbonate suivie de la réduction du produit obtenu au moyen d'un hydrure de trialcoylétain, suivi éventuellement du remplacement du groupement protecteur représenté par Z₁ ou R₈ par un atome d'hydrogène dans les conditions de la revendication 6.

12. Procédé de préparation d'un produit selon l'une des revendications 1, 2 ou 3 **caractérisé en ce que** l'on estérifie un produit selon l'une des revendications 1, 2 ou 3 au moyen d'un acide de formule générale : 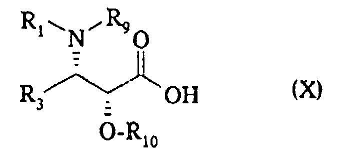 dans laquelle, ou bien R₉ représente un atome d'hydrogène et R₁₀ représente un groupement protecteur de la fonction hydroxy, ou bien R₉ et R₁₀ forment ensemble un hétérocycle saturé à 5 ou 6 chaînons, ou d'un dérivé de cet acide, puis remplace les groupements protecteurs par des atomes d'hydrogène en opérant dans des conditions connues.

13. Composition pharmaceutique **caractérisée en ce qu'**elle contient au moins un produit selon l'une des revendications 1, 2 ou 3 pour lequel Z représente un radical de formule générale (II) en association avec un ou plusieurs diluants ou adjuvants pharmaceutiquement acceptables et éventuellement un ou plusieurs composés compatibles et pharmacologiquement actifs.

## Patentansprüche

1. Neue Taxoide der allgemeinen Formel (I) 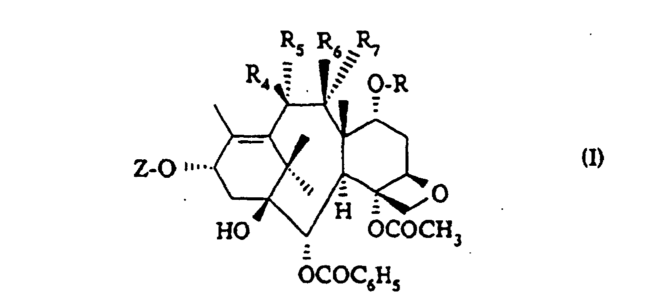 in der
Z ein Wasserstoffatom oder einen Rest der allgemeinen Formel (II) darstellt, worin 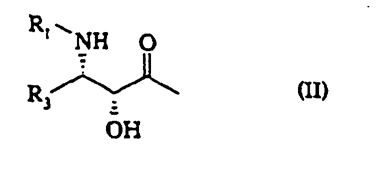 R₁ einen Rest Benzoyl bedeutet, gegebenenfalls substituiert durch ein oder mehrere, gleiche oder verschiedene Atome oder Reste, ausgewählt unter den Halogenatomen und den Resten Alkyl mit 1 bis 4 Kohlenstoffatomen, Alkoxy mit 1 bis 4 Kohlenstoffatomen oder Trifluormethyl, Thenoyl oder Furoyl oder einem Rest R₂-O-CO-, worin R₂ einen Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen, Bicycloalkyl mit 7 bis 10 Kohlenstoffatomen darstellt, wobei diese Reste gegebenenfalls substituiert sind durch ein oder mehrere Substituenten, ausgewählt unter den Halogenatomen und den Resten Hydroxy, Alkoxy mit 1 bis 4 Kohlenstoffatomen, Dialkylamino, bei dem jeder Teil Alkyl 1 bis 4 Kohlenstoffatome enthält, Piperidino, Morpholino, 1-Piperazinyl (gegebenenfalls substituiert in -4 durch einen Rest Alkyl mit 1 bis 4 Kohlenstoffatomen oder durch einen Rest Phenylalkyl, dessen Teil Alkyl 1 bis 4 Kohlenstoffatome enthält), Cycloalkyl mit 3 bis 6 Kohlenstoffatomen, Cycloalkenyl mit 4 bis 6 Kohlenstoffatomen, Phenyl (gegebenenfalls substituiert durch ein oder mehrere Atome oder Reste, ausgewählt unter den Halogenatomen und den Resten Alkyl mit 1 bis 4 Kohlenstoffatomen oder Alkoxy mit 1 bis 4 Kohlenstoffatomen), Cyano, Carboxy oder Alkoxycarbonyl, dessen Teil Alkyl 1 bis 4 Kohlenstoffatome enthält, einem Rest Phenyl oder α- oder β-Naphthyl, gegebenenfalls substituiert durch ein oder mehrere Atome oder Reste, ausgewählt unter den Halogenatomen und den Resten Alkyl mit 1 bis 4 Kohlenstoffatomen oder Alkoxy mit 1 bis 4 Kohlenstoffatomen, oder einem aromatischen heterocyclischen Rest mit 5 Ringgliedern, vorzugsweise ausgewählt unter den Resten Furyl und Thienyl, oder einem gesättigten heterocyclischen Rest mit 4 bis 6 Kohlenstoffatomen, gegebenenfalls substituiert durch einen oder mehrere Reste Alkyl mit 1 bis 4 Kohlenstoffatomen,
R₃ einen geraden oder verzweigten Rest Alkyl mit 1 bis 8 Kohlenstoffatomen, einen geraden oder verzweigten Rest Alkenyl mit 2 bis 8 Kohlenstoffatomen, einen geraden oder verzweigten Rest Alkinyl mit 2 bis 8 Kohlenstoffatomen, Cycloalkyl mit 3 bis 6 Kohlenstoffatomen, Cycloalkenyl mit 4 bis 6 Kohlenstoffatomen, Phenyl oder α- oder β-Naphthyl darstellt, gegebenenfalls substituiert durch ein oder mehrere Atome oder Reste, ausgewählt unter den Halogenatomen und den Resten Alkyl, Alkenyl, Alkinyl, Aryl, Aralkyl, Alkoxy, Alkylthio, Aryloxy, Arylthio, Hydroxy, Hydroxyalkyl, Mercapto, Formyl, Acyl, Acylamino, Aroylamino, Alkoxycarbonylamino, Amino, Alkylamino, Dialkylamino, Carboxy, Alkoxycarbonyl, Carbamoyl, Alkylcarbamoyl, Dialkylcarbamoyl, Cyano, Nitro und Trifluormethyl, oder einen aromatischen Heterocyclus mit 5 Ringgliedern und einem oder mehreren, gleichen oder verschiedenen Heteroatomen bedeutet, ausgewählt unter den Atomen von Stickstoff, Sauerstoff oder Schwefel und gegebenenfalls substituiert durch einen oder mehrere, gleiche oder verschiedene Substituenten, ausgewählt unter den Halogenatomen und den Resten Alkyl, Aryl, Amino, Alkylamino, Dialkylamino, Alkoxycarbonylamino, Acyl, Arylcarbonyl, Cyano, Carboxy, Carbamoyl, Alkylcarbamoyl, Dialkylcarbamoyl oder Alkoxycarbonyl, mit der Maßgabe, daß in den Substituenten der Reste Phenyl, α- oder β-Naphthyl und der aromatischen Heterocyclen die Reste Alkyl und die Teile Alkyl der anderen Reste 1 bis 4 Kohlenstoffatome enthalten und daß die Reste Alkenyl und Alkinyl 2 bis 8 Kohlenstoffatome enthalten und daß die Reste Aryl Reste Phenyl oder α- oder β-Naphthyl sind,
R₄ entweder ein Wasserstoffatom ist,
R₆ und R₇ zusammen eine Ketonfunktion bilden, und
R und R₅ zusammen eine Bindung bilden,
oder R₄ ein Wasserstoffatom oder einen Rest Hydroxy oder einen Rest Alkoxy mit 1 bis 6 Kohlenstoffatomen in gerader oder verzweigter Kette, Alkenyloxy mit 3 bis 6 Kohlenstoffatomen in gerader oder verzweigter Kette, Alkinyloxy mit 3 bis 6 Kohlenstoffatomen in gerader oder verzweigter Kette, Cycloalkyloxy mit 3 bis 6 Kohlenstoffatomen, Cycloalkenyloxy mit 3 bis 6 Kohlenstoffatomen, Alkanoyloxy, dessen Teil Alkanoyl 1 bis 6 Kohlenstoffatome in gerader oder verzweigter Kette enthält, Aroyloxy, dessen Teil Aryl 6 bis 10 Kohlenstoffatome enthält, Alkenoyloxy, dessen Teil Alkenoyl 3 bis 6 Kohlenstoffatome in gerader oder verzweigter Kette enthält, Alkinoyloxy, dessen Teil Alkinoyl 3 bis 6 Kohlenstoffatome in gerader oder verzweigter Kette enthält, Cycloalkanoyloxy mit 3 bis 6 Kohlenstoffatomen, Alkoxyacetyl, dessen Teil Alkyl 1 bis 6 Kohlenstoffatome in gerader oder verzweigter Kette enthält, Alkylthioacetyl, dessen Teil Alkyl 1 bis 6 Kohlenstoffatome in gerader oder verzweigter Kette enthält, Alkyloxycarbonyloxy, dessen Teil Alkyl 1 bis 6 Kohlenstoffatome in gerader oder verzweigter Kette enthält, darstellt, wobei diese Reste gegebenenfalls substituiert sind durch ein oder mehrere Halogenatome oder durch einen Rest Alkoxy mit 1 bis 4 Kohlenstoffatomen, Alkylthio mit 1 bis 4 Kohlenstoffatomen, oder einen Rest Carboxy, Alkyloxycarbonyl, dessen Teil Alkyl 1 bis 4 Kohlenstoffatomen enthält, Cyano, Carbamoyl, N-Alkylcarbamoyl oder N,N-Dialkylcarbamoyl, bei dem jeder Teil Alkyl 1 bis 4 Kohlenstoffatome enthält oder zusammen mit dem Stickstoffatom, an das sie gebunden sind, einen gesättigten heterocyclischen Rest mit 5 oder 6 Ringgliedern und gegebenenfalls einem zweiten Heteroatom bilden, ausgewählt unter den Atomen von Sauerstoff, Schwefel oder Stickstoff und gegebenenfalls substituiert durch einen Rest Alkyl mit 1 bis 4 Kohlenstoffatomen oder einen Rest Phenyl oder einen Rest Phenylalkyl, dessen Teil Alkyl 1 bis 4 Kohlenstoffatome enthält, oder R₄ auch einen Rest Carbamoyloxy, Alkylcarbamoyloxy, dessen Teil Alkyl 1 bis 4 Kohlenstoffatome enthält, Dialkylcarbamoyloxy, bei dem jeder Teil Alkyl 1 bis 4 Kohlenstoffatome enthält, oder einen Rest Benzoyloxy oder Heterocyclylcarbonyloxy bedeutet, bei dem der heterocyclische Teil einen aromatischen Heterocyclus mit 5 oder 6 Ringgliedern darstellt, der ein oder mehrere Heteroatome enthält, ausgewählt unter den Atomen von Sauerstoff, Schwefel oder Stickstoff,
R₅ ein Wasserstoffatom bedeutet,
oder R₄ und R₅ zusammen eine Ketonfunktion bilden,
R₆ ein Wasserstoffatom ist und
R und R₇ zusammen eine Bindung bilden.

2. Neue Taxoide nach Anspruch 1, worin Z ein Wasserstoffatom oder einen Rest der allgemeinen Formel (II) darstellt, in der R₁ einen Rest Benzoyl oder einen Rest R₂-O-CO- bedeutet, worin R₂ ein Rest tert.-Butyl ist und R₃ einen Rest Alkyl mit 1 bis 6 Kohlenstoffatomen, Alkenyl mit 2 bis 6 Kohlenstoffatomen, Cycloalkyl mit 3 bis 6 Kohlenstoffatomen, Phenyl, gegebenenfalls substituiert durch ein oder mehrere, gleiche oder verschiedene Atome oder Reste, ausgewählt unter den Atomen und den Resten Alkyl, Alkoxy, Dialkylamino, Acylamino, Alkoxycarbonylamino oder Trifluormethyl, oder einen Rest 2-Furyl oder 3-Furyl, 2-Thienyl oder 3-Thienyl oder 2-Thiazolyl, 4-Thiazolyl oder 5-Thiazolyl darstellt, oder R₄ entweder ein Wasserstoffatom ist, R₆ und R₇ zusammen eine Ketonfunktion bilden und R und R₅ zusammen eine Bindung bilden, oder R₄ einen Rest Hydroxy, Alkoxy mit 1 bis 6 Kohlenstoffatomen, Alkanoyloxy mit 1 bis 6 Kohlenstoffatomen oder Alkoxyacetyl darstellt, bei dem jeder Teil Alkyl 1 bis 6 Kohlenstoffatome enthält, R₅ ein Wasserstoffatom ist, R₆ ein Wasserstoffatom ist und R und R₇ zusammen eine Bindung bilden, oder auch R₄ und R₅ zusammen eine Ketonfunktion bilden, R₆ ein Wasserstoffatom ist, R und R₇ zusammen eine Bindung bilden.

3. Neue Taxoide nach Anspruch 1, worin Z ein Wasserstoffatom oder einen Rest der allgemeinen Formel (II) darstellt, in der R₁ einen Rest Benzoyl oder einen Rest R₂-O-CO- bedeutet, worin R₂ ein Rest tert.-Butyl ist und R₃ einen Rest Isobutyl, Isobutenyl, Butenyl, Cyclohexyl, Phenyl, 2-Furyl, 3-Furyl, 2-Thienyl, 3-Thienyl, 2-Thiazolyl, 4-Thiazolyl oder 5-Thiazolyl darstellt, oder R₄ entweder ein Wasserstoffatom ist, R₆ und R₇ zusammen eine Ketonfunktion bilden und R und R₅ zusammen eine Bindung bilden, oder R₄ einen Rest Hydroxy oder einen Rest Methoxy, Acetoxy, Propanoyloxy oder Methoxyacetoxy darstellt, R₅ ein Wasserstoffatom ist, R₆ ein Wasserstoffatom ist und R und R₇ zusammen eine Bindung bilden.

4. Verfahren zur Herstellung eines Produktes nach einem der Ansprüche 1, 2 oder 3, worin R₄ entweder ein Wasserstoffatom ist, R₆ und R₇ zusammen eine Ketonfunktion bilden und R und R₅ zusammen eine Bindung bilden, oder R₄ einen Rest Hydroxy darstellt, R₅ ein Wasserstoffatom ist, R₆ ein Wasserstoffatom ist und R und R₇ zusammen eine Bindung bilden, **dadurch gekennzeichnet, daß** man ein Reduktionsmittel mit einem Produkt der allgemeinen Formel (III) 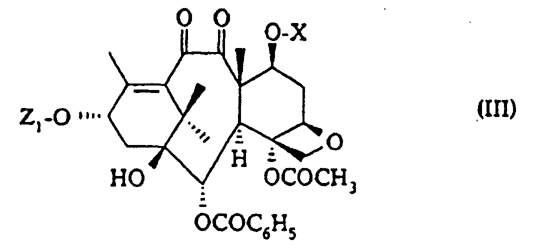 zur Reaktion bringt, in der Z₁ ein Wasserstoffatom oder eine Schutzgruppe für die Hydroxyfunktion oder einen Rest der allgemeinen Formel (IV) 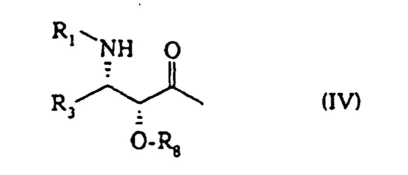 darstellt, worin R₁ und R₃ wie in einem der Ansprüche 1, 2 oder 3 definiert sind und R₈ eine Schutzgruppe für die Hydroxyfunktion bedeutet und X mit dem Sauerstoff, an das es gebunden ist, eine Abgangsgruppe darstellt, ausgewählt unter den Resten Alkylsulfonyl mit 1 bis 4 Kohlenstoffatomen, gegebenenfalls substituiert durch ein oder mehrere Halogenatome, Arylsulfonyl, dessen Teil Aryl ein Rest Phenyl ist, gegebenenfalls substituiert durch ein oder mehrere, gleiche oder verschiedene Atome oder Reste, ausgewählt unter den Halogenatomen und den Resten Alkyl mit 1 bis 4 Kohlenstoffatomen, Nitro oder Trifluormethyl, um ein Produkt der allgemeinen Formel (V) 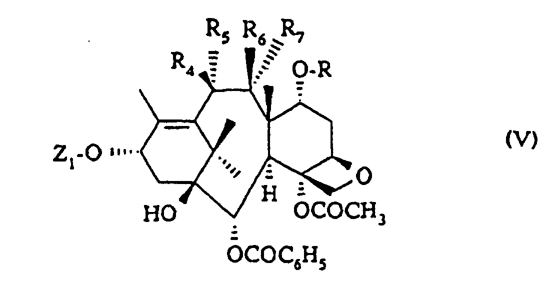 in der Z₁, R, R₄, R₅, R₆ und R₇ wie vorstehend definiert sind, in Form einer Mischung eines Produktes der allgemeinen Formel (I), in der R₄ ein Wasserstoffatom ist, R₆ und R₇ zusammen eine Ketonfunktion bilden und R und R₅ zusammen eine Bindung bilden, und eines Produktes der allgemeinen Formel (I), in der R₄ einen Rest Hydroxy darstellt, R₅ ein Wasserstoffatom ist, R₆ ein Wasserstoffatom ist, und R und R₇ zusammen eine Bildung bilden, zu erhalten, das man nach üblichen Methoden trennt und anschließend gegebenenfalls die durch Z₁ oder R₈ verkörperten Schutzgruppen durch ein Wasserstoffatom austauscht.

5. Verfahren nach Anspruch 4, **dadurch gekennzeichnet, daß** das Reduktionsmittel ausgewählt wird unter den Aluminiumhydriden und den Borhydriden wie den Borhydriden von Alkalimetallen oder Erdalkalimetallen in Anwesenheit eines aliphatischen Alkohols mit 1 bis 4 Kohlenstoffatomen wie Ethanol, wobei die Reaktion bei einer Temperatur zwischen 0 °C und 50 °C durchgeführt wird.

6. Verfahren nach Anspruch 4, **dadurch gekennzeichnet, daß** man in dem Fall, wo die Schutzgruppe einen Rest Silyl darstellt, diesen mit Hilfe einer Mineralsäure in einem aliphatischen Alkohol mit 1 bis 3 Kohlenstoffatomen durch ein Wasserstoffatom austauscht, wobei man bei einer Temperatur zwischen -10 °C und 20 °C arbeitet, oder auch mit Hilfe eines Komplexes Fluorwasserstoffsäure/Triethylamin in einem inerten organischen Lösungsmittel und bei einer Temperatur zwischen 0 °C und 50 °C.

7. Verfahren zur Herstellung eines Produktes nach einem der Ansprüche 1, 2 oder 3, worin Z wie in einem der Ansprüche 1, 2 oder 3 definiert ist, R₄ einen Rest Alkoxy mit 1 bis 6 Kohlenstoffatomen in gerader oder verzweigter Kette, Alkenyloxy mit 3 bis 6 Kohlenstoffatomen in gerader oder verzweigter Kette, Alkinyloxy mit 3 bis 6 Kohlenstoffatomen in gerader oder verzweigter Kette, Cycloalkyloxy mit 3 bis 6 Kohlenstoffatomen, Cycloalkenyloxy mit 3 bis 6 Kohlenstoffatomen, Alkanoyloxy, dessen Teil Alkanoyl 1 bis 6 Kohlenstoffatome in gerader oder verzweigter Kette enthält, Alkenoyloxy, dessen Teil Alkenoyl 3 bis 6 Kohlenstoffatome in gerader oder verzweigter Kette enthält, Alkinoyloxy, dessen Teil Alkinoyl 3 bis 6 Kohlenstoffatome in gerader oder verzweigter Kette enthält, Cycloalkanoyloxy mit 1 bis 6 Kohlenstoffatomen, Alkoxyacetyl, dessen Teil Alkyl 1 bis 6 Kohlenstoffatome in gerader oder verzweigter Kette enthält, Alkylthioacetyl, dessen Teil Alkyl 1 bis 6 Kohlenstoffatome in gerader oder verzweigter Kette enthält, Alkyloxycarbonyloxy, dessen Teil Alkyl 1 bis 6 Kohlenstoffatome in gerader oder verzweigter Kette enthält, darstellt, wobei diese Reste gegebenenfalls substituiert sind durch ein oder mehrere Halogenatome oder durch einen Rest Alkoxy mit 1 bis 4 Kohlenstoffatomen, Alkylthio mit 1 bis 4 Kohlenstoffatomen, oder einen Rest Carboxy, Alkyloxycarbonyl, dessen Teil Alkyl 1 bis 4 Kohlenstoffatomen enthält, Cyano, Carbamoyl, N-Alkylcarbamoyl oder N,N-Dialkylcarbamoyl, bei dem jeder Teil Alkyl 1 bis 4 Kohlenstoffatome enthält oder zusammen mit dem Stickstoffatom, an das sie gebunden sind, einen gesättigten heterocyclischen Rest mit 5 oder 6 Ringgliedern und gegebenenfalls einem zweiten Heteroatom bilden, ausgewählt unter den Atomen von Sauerstoff, Schwefel oder Stickstoff und gegebenenfalls substituiert durch einen Rest Alkyl mit 1 bis 4 Kohlenstoffatomen oder einen Rest Phenyl oder einen Rest Phenylalkyl, dessen Teil Alkyl 1 bis 4 Kohlenstoffatome enthält, oder R₄ auch einen Rest Carbamoyloxy, Alkylcarbamoyloxy, dessen Teil Alkyl 1 bis 4 Kohlenstoffatome enthält, oder Dialkylcarbamoyloxy, bei dem jeder Teil Alkyl 1 bis 4 Kohlenstoffatome enthält oder einen Rest Benzoyloxy oder Heterocyclylcarbonyloxy bedeutet, bei dem der heterocyclische Teil einen aromatischen Heterocyclus mit 5 oder 6 Ringgliedern darstellt, der ein oder mehrere Heteroatome enthält, ausgewählt unter den Atomen von Sauerstoff, Schwefel oder Stickstoff, R₅ ein Wasserstoffatom ist, R₆ ein Wasserstoffatom ist, und R und R₇ zusammen eine Bindung bilden, **dadurch gekennzeichnet, daß** man ein Produkt der allgemeinen Formel (IX)
R'₄-Y (IX)
in der R'₄ so wie R'₄-O- ist und identisch mit R₄ wie vorstehend definiert und Y eine Abgangsgruppe darstellt, mit einem Produkt der allgemeinen Formel (V) 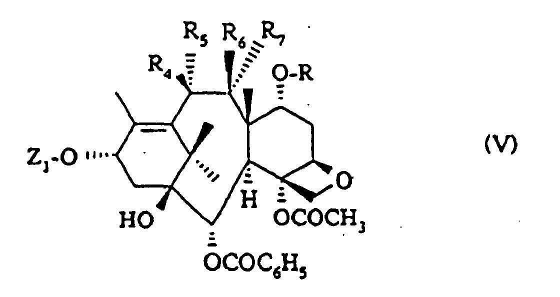 zur Reaktion bringt, in der Z₁ wie vorstehend definiert ist, R₄ einen Rest Hydroxy darstellt, R₆ ein Wasserstoffatom ist und R und R₇ zusammen eine Bindung bilden, gefolgt vom Austausch der durch Z₁ oder R₈ verkörperten Schutzgruppe durch ein Wasserstoffatom unter den Bedingungen von Anspruch 6.

8. Verfahren nach Anspruch 7, **dadurch gekennzeichnet, daß** man vor der Einwirkung des Produktes der allgemeinen Formel (IX) die Hydroxyfunktion in Position 10 gegebenenfalls mit Hilfe eines Hydrids, eines Amids oder eines Alkylids von Alkalimetall metalliert.

9. Verfahren zur Herstellung eines Produktes nach einem der Ansprüche 1, 2 oder 3, worin Z einen Rest der allgemeinen Formel (II) darstellt, R₄ und R₅ zusammen eine Ketonfunktion bilden, R₆ ein Wasserstoffatom ist und R und R₇ zusammen eine Bindung bilden, **dadurch gekennzeichnet, daß** man ein Produkt der allgemeinen Formel (V) 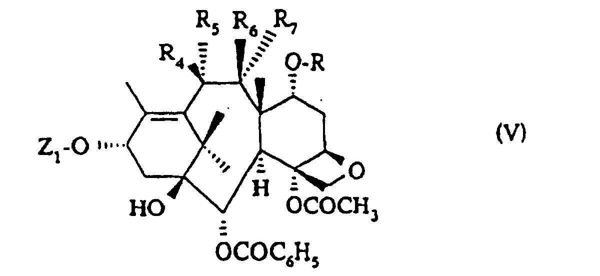 oxidiert, in der Z₁ wie in Anspruch 4 definiert ist, R₄ einen Rest Hydroxy darstellt, R₅ ein Wasserstoffatom ist, R₆ ein Wasserstoffatom ist und R und R₇ zusammen eine Bindung bilden, und man anschließend gegebenenfalls die durch Z₁ oder R₈ verkörperte Schutzgruppe durch ein Wasserstoffatom unter den Bedingungen von Anspruch 6 austauscht.

10. Verfahren nach Anspruch 9, **dadurch gekennzeichnet, daß** die Oxidation mit Hilfe von Sauerstoff, Ammonium-perruthenat, Mangandioxid, Kupferacetat oder Pyridinium-chlorchromat realisiert wird.

11. Verfahren zur Herstellung eines Produktes nach einem der Ansprüche 1, 2 oder 3, worin Z einen Rest der allgemeinen Formel (II) darstellt, R₄, R₅ und R₆ jeweils ein Wasserstoffatom bedeuten und R und R₇ zusammen eine Bindung bilden, wobei das Produkt ausgehend von einem Produkt der allgemeinen Formel (V), in der Z₁ wie in Anspruch 4 definiert ist, R₄ einen Rest Hydroxy darstellt, R₅ und R₆ jeweils ein Wasserstoffatom bedeuten und R und R₇ zusammen eine Bindung bilden, nach Umwandlung des durch R₄ verkörperten Restes Hydroxy in Dithiocarbonat, gefolgt von der Reduktion des erhaltenen Produktes mit Hilfe eines Trialkylzinn-hydrids und gegebenenfalls gefolgt von dem Austausch der durch Z₁ oder R₈ verkörperten Schutzgruppe durch ein Wasserstoffatom unter den Bedingungen von Anspruch 6 erhalten werden kann.

12. Verfahren zur Herstellung eines Produktes nach einem der Ansprüche 1, 2 oder 3, **dadurch gekennzeichnet, daß** man ein Produkt nach einem der Ansprüche 1, 2 oder 3 mit Hilfe einer Säure der allgemeinen Formel (X) 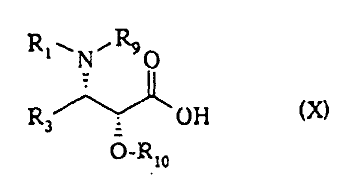 in der entweder R₉ ein Wasserstoffatom ist und R₁₀ eine Schutzgruppe für die Hydroxyfunktion darstellt, oder R₉ und R₁₀ zusammen einen gesättigten Heterocyclus mit 5 oder 6 Ringgliedern bilden, oder einem Derivat dieser Säure verestert und anschließend die Schutzgruppen durch Wasserstoffatome austauscht, wobei man unter bekannten Bedingungen arbeitet.

13. Pharmazeutische Zusammensetzung, **dadurch gekennzeichnet, daß** sie mindestens ein Produkt nach einem der Ansprüche 1, 2 oder 3, worin Z einen Rest der Formel (II) darstellt, in Assoziation mit einem oder mehreren pharmazeutisch akzeptablen Verdünnungsmitteln oder Zusatzstoffen und gegebenenfalls einer oder mehreren kompatiblen und pharmakologisch aktiven Verbindungen enthält.

## Claims

1. New taxoids of general formula: 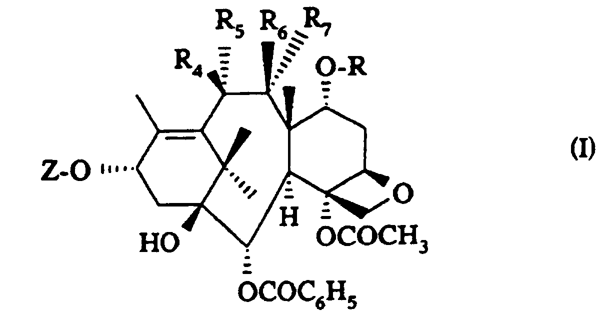 in which:
Z represents a hydrogen atom or a radical of general formula: 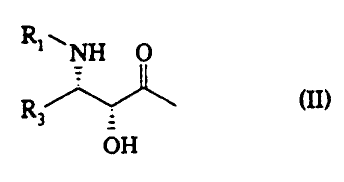 in which:
R₁ represents a benzoyl radical optionally substituted with one or more identical or different atoms or radicals chosen from halogen atoms and alkyl radicals containing 1 to 4 carbon atoms, alkoxy radicals containing 1 to 4 carbon atoms or a trifluoromethyl radical, a thenoyl radical or a furoyl radical or a radical R₂-O-CO- in which R₂ represents an alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals being optionally substituted with one or more substituents chosen from halogen atoms and hydroxyl radicals, alkoxy radicals containing 1 to 4 carbon atoms, dialkylamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl portion contains 1 to 4 carbon atoms, a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms, or a 5-membered aromatic heterocyclic radical preferably chosen from furyl and thienyl radicals, or a saturated heterocyclic radical containing 4 to 6 carbon atoms, optionally substituted with one or more alkyl radicals containing 1 to 4 carbon atoms,
R₃ represents an unbranched or branched alkyl radical containing 1 to 8 carbon atoms, an unbranched or branched alkenyl radical containing 2 to 8 carbon atoms, an unbranched or branched alkynyl radical containing 2 to 8 carbon atoms, a cycloalkyl radical containing 3 to 6 carbon atoms, cycloalkenyl radicals containing 4 to 6 carbon atoms, a phenyl or α- or β-naphthyl radical optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals, or a 5-membered aromatic heterocycle containing one or more identical or different hetero atoms chosen from nitrogen, oxygen and sulphur atoms and optionally substituted with one or more identical or different substituents chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl or alkoxycarbonyl radicals, on the understanding that, in the substituents of the phenyl, α- or β-naphthyl and aromatic heterocyclic radicals, the alkyl radicals and the alkyl portions of the other radicals contain 1 to 4 carbon atoms, and that the alkenyl and alkynyl radicals contain 2 to 8 carbon atoms, and that the aryl radicals are phenyl or α- or β-naphthyl radicals,
either R₄ represents a hydrogen atom,
R₆ and R₇ together form a ketone function, and
R and R₅ together form a bond,
or R₄ represents a hydrogen atom or a hydroxyl radical or an alkoxy radical containing 1 to 6 carbon atoms in an unbranched or branched chain, an alkenyloxy radical containing 3 to 6 carbon atoms in an unbranched or branched chain, an alkynyloxy radical containing 3 to 6 carbon atoms in an unbranched or branched chain, a cycloalkyloxy radical containing 3 to 6 carbon atoms, a cycloalkenyloxy radical containing 3 to 6 carbon atoms, an alkanoyloxy radical in which the alkanoyl portion contains 1 to 6 carbon atoms in an unbranched or branched chain, an aroyloxy radical in which the aryl portion contains 6 to 10 carbon atoms, an alkenoyloxy radical in which the alkenoyl portion contains 3 to 6 carbon atoms in an unbranched or branched chain, an alkynoyloxy radical in which the alkynoyl portion contains 3 to 6 carbon atoms in an unbranched or branched chain, a cycloalkanoyloxy radical containing 3 to 6 carbon atoms, an alkoxyacetyl radical in which the alkyl portion contains 1 to 6 carbon atoms in an unbranched or branched chain, an alkylthioacetyl radical in which the alkyl portion contains 1 to 6 carbon atoms in an unbranched or branched chain or an alkyloxycarbonyloxy radical in which the alkyl portion contains 1 to 6 carbon atoms in an unbranched or branched chain, these radicals being optionally substituted with one or more halogen atoms or with an alkoxy radical containing 1 to 4 carbon atoms, an alkylthio radical containing 1 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur and nitrogen atoms, optionally substituted with an alkyl radical containing 1 to 4 carbon atoms or a phenyl radical or a phenylalkyl radical in which the alkyl portion contains 1 to 4 carbon atoms, or alternatively R₄ represents a carbamoyloxy radical or an alkylcarbamoyloxy radical in which the alkyl portion contains 1 to 4 carbon atoms, a dialkylcarbamoyloxy radical in which each alkyl portion contains 1 to 4 carbon atoms or a benzoyloxy radical or a heterocyclylcarbonyloxy radical in which radical the heterocyclic portion represents a 5- or 6-membered aromatic heterocycle containing one or more hetero atoms chosen from oxygen, sulphur and nitrogen atoms,
R₅ represents a hydrogen atom,
or R₄ and R₅ together form a ketone function,
R₆ represents a hydrogen atom, and
R and R₇ together form a bond.

2. New taxoids according to Claim 1, for which Z represents a hydrogen atom or a radical of general formula (II) in which R₁ represents a benzoyl radical or a radical R₂-O-CO- in which R₂ represents a tert-butyl radical and R₃ represents an alkyl radical containing 1 to 6 carbon atoms, an alkenyl radical containing 2 to 6 carbon atoms, a cycloalkyl radical containing 3 to 6 carbon atoms, a phenyl radical optionally substituted with one or more identical or different atoms or radicals chosen from halogen atoms and alkyl, alkoxy, dialkylamino, acylamino, alkoxycarbonylamino or trifluoromethyl radicals, or a 2- or 3-furyl, 2- or 3-thienyl or 2-, 4- or 5-thiazolyl radical, and either R₄ represents a hydrogen atom, R₆ and R₇ together form a ketone function and R and R₅ together form a bond, or R₄ represents a hydroxyl radical or an alkoxy radical containing 1 to 6 carbon atoms, an alkanoyloxy radical containing 1 to 6 carbon atoms or an alkoxyacetyl radical in which the alkyl portion contains 1 to 6 carbon atoms, R₅ represents a hydrogen atom, R₆ represents a hydrogen atom and R and R₇ together form a bond, or R₄ and R₅ together form a ketone function, R₆ represents a hydrogen atom, R and R₇ together form a bond.

3. New taxoids according to Claim 1, for which Z represents a hydrogen atom or a radical of general formula (II) in which R₁ represents a benzoyl radical or a radical R₂-O-CO- in which R₂ represents a tert-butyl radical and R₃ represents an isobutyl, isobutenyl, butenyl, cyclohexyl, phenyl, 2-furyl, 3-furyl, 2-thienyl, 3-thienyl, 2-thiazolyl, 4-thiazolyl or 5-thiazolyl radical, either R₄ represents a hydrogen atom, R₆ and R₇ together form a ketone function and R and R₅ together form a bond, or R₄ represents a hydroxyl radical or a methoxy, acetoxy, propanoyloxy or methoxyacetoxy radical, R₅ represents a hydrogen atom, R₆ represents a hydrogen atom and R and R₇ together form a bond.

4. Process for the preparation of a product according to one of Claims 1, 2 or 3, in which either R₄ represents a hydrogen atom, R₆ and R₇ together form a ketone function and R and R₅ together form a bond, or R₄ represents a hydroxyl radical, R₅ represents a hydrogen atom, R₆ represents a hydrogen atom and R and R₇ together form a bond, **characterized in that** a reducing agent is reacted with a product of general formula: 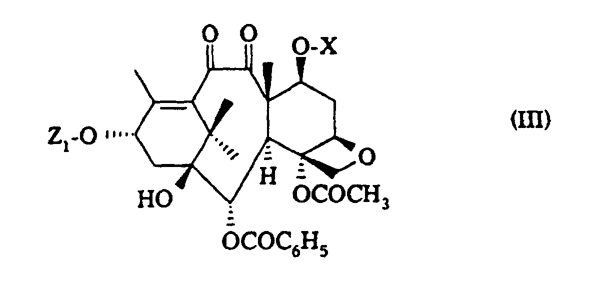 in which Z₁ represents a hydrogen atom or a group protecting the hydroxyl function or a radical of general formula: 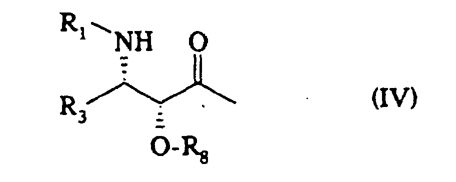 in which R₁ and R₃ are defined as in one of Claims 1, 2 or 3 and R₈ represents a group protecting the hydroxyl function, and X represents, with the oxygen atom to which it is linked, a leaving group chosen from alkylsulphonyl radicals containing 1 to 4 carbon atoms optionally substituted with one or more halogen atoms, or arylsulphonyl radicals in which the aryl portion is a phenyl radical optionally substituted with one or more identical or different atoms or radicals chosen from halogen atoms and alkyl radicals containing 1 to 4 carbon atoms, or nitro or trifluoromethyl radicals, to obtain a product of general formula: 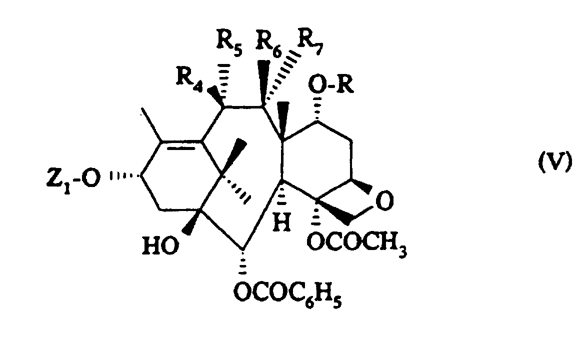 in which Z₁, R, R₄, R₅, R₆ and R₇ are defined as above, in the form of a mixture of a product of general formula (I) in which R₄ represents a hydrogen atom, R₆ and R₇ together form a ketone function, and R and R₅ together form a bond, and of a product of general formula (I) in which R₄ represents a hydroxyl radical, R₅ represents a hydrogen atom, R₆ represents a hydrogen atom, and R and R₇ together form a bond, which is separated by the usual methods, and then the protective group represented by Z₁ or R₈ is optionally replaced with a hydrogen atom.

5. Process according to Claim 4, **characterized in that** the reducing agent is chosen from aluminohydrides or borohydrides such as alkali or alkaline-earth metal borohydrides, in the presence of an aliphatic alcohol containing 1 to 4 carbon atoms such as ethanol, the reaction being carried out at a temperature of between 0 and 50°C.

6. Process according to Claim 4, **characterized in that** when the protective group represents a silylated radical, it is replaced by a hydrogen atom by means of an inorganic acid in an aliphatic alcohol containing 1 to 3 carbon atoms at a temperature of between -10 and 20°C or by means of the hydrofluoric acid/triethylamine complex, working in an inert organic solvent at a temperature of between 0 and 50°C.

7. Process for the preparation of a product according to one of Claims 1, 2 or 3, for which Z is defined as in one of Claims 1, 2 or 3, R₄ represents an alkoxy radical containing 1 to 6 carbon atoms in an unbranched or branched chain, an alkenyloxy radical containing 3 to 6 carbon atoms in an unbranched or branched chain, an alkynyloxy radical containing 3 to 6 carbon atoms in an unbranched or branched chain, a cycloalkyloxy radical containing 3 to 6 carbon atoms, a cycloalkenyloxy radical containing 3 to 6 carbon moms, an alkanoyloxy radical in which the alkanoyl portion contains 1 to 6 carbon atoms in an unbranched or branched chain, an alkenoyloxy radical in which the alkenoyl portion contains 3 to 6 carbon atoms in an unbranched or branched chain, an alkynoyloxy radical in which the alkynoyl portion contains 3 to 6 carbon atoms in an unbranched or branched chain, a cycloalkanoyloxy radical containing 1 to 6 carbon atoms, an alkoxyacetyl radical in which the alkyl portion contains 1 to 6 carbon atoms in an unbranched or branched chain, an alkylthioacetyl radical in which the alkyl portion contains 1 to 6 carbon atoms in an unbranched or branched chain or an alkyloxycarbonyloxy radical in which the alkyl portion contains 1 to 6 carbon atoms in an unbranched or branched chain, these radicals being optionally substituted with one or more halogen atoms or with an alkoxy radical containing 1 to 4 carbon atoms, an alkylthio radical containing 1 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur and nitrogen atoms, optionally substituted with an alkyl radical containing 1 to 4 carbon atoms or a phenyl radical or a phenylalkyl radical in which the alkyl portion contains 1 to 4 carbon atoms, or alternatively R₄ represents a carbamoyloxy radical or an alkylcarbamoyloxy radical in which the alkyl portion contains 1 to 4 carbon atoms, a dialkylcarbamoyloxy radical in which each alkyl portion contains 1 to 4 carbon atoms or a benzoyloxy radical or a heterocyclylcarbonyloxy radical in which radical the heterocyclic portion represents a 5- or 6-membered aromatic heterocycle containing one or more hetero atoms chosen from oxygen, sulphur and nitrogen atom, R₅ represents a hydrogen atom, R₆ represents a hydrogen atom, and R and R₇ together form a bond, **characterized in that** a product of general formula:
R'₄-Y (IX)
in which R'₄ is such that R'₄-O- is identical to R₄ defined as above and Y represents a leaving group is reacted with a product of general formula: 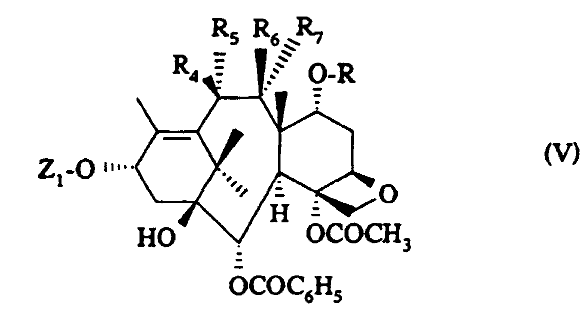 in which Z₁ is defined as above, R₄ represents a hydroxyl radical, R₆ represents a hydrogen atom and R and R₇ together form a bond, followed by the replacement of the protective group represented by Z₁ or R₈ by a hydrogen atom under the conditions of Claim 6.

8. Process according to Claim 7, **characterized in that** prior to the action of the product of general formula (IX) the hydroxyl function at position 10 is optionally metalated by means of an alkali metal hydride, amide or alkylide.

9. Process for the preparation of a product according to one of Claims 1, 2 or 3, for which Z represents a radical of general formula (II), R₄ and R₅ together form a ketone function, R₆ represents a hydrogen atom, and R and R₇ together form a bond, **characterized in that** a product of general formula: 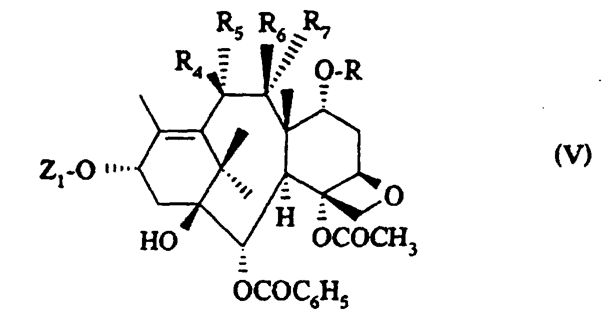 in which Z₁ is defined as in Claim 4, R₄ represents a hydroxyl radical, R₅ represents a hydrogen atom, R₆ represents a hydrogen atom and R and R₇ together form a bond, is oxidized and then the protective group represented by Z₁ or R₈ is optionally replaced by a hydrogen atom under the conditions of Claim 6.

10. Process according to Claim 9, **characterized in that** the oxidation is carried out by means of oxygen, ammonium peruthenate, manganese dioxide, copper acetate or pyridinium chlorochromate.

11. Process for the preparation of a product according to one of Claims 1, 2 or 3, for which Z represents a radical of general formula (II), R₄, R₅ and R₆ each represent a hydrogen atom and R and R₇ together form a bond, may be obtained from a product of general formula (V) in which Z₁ is defined as in Claim 4, R₄ represents a hydroxyl radical, R₅ and R₆ each represent a hydrogen atom and R and R₇ together form a bond, after conversion of the hydroxyl radical represented by R₄ to a dithiocarbonate followed by the reduction of the product obtained by means of a trialkyltin hydride optionally followed by the replacement of the protective group represented by Z₁ or R₈ by a hydrogen atom under the conditions of Claim 6.

12. Process for the preparation of a product according to one of Claims 1, 2 or 3, **characterized in that** a product according to one of Claims 1, 2 or 3 is esterified by means of an acid of general formula: 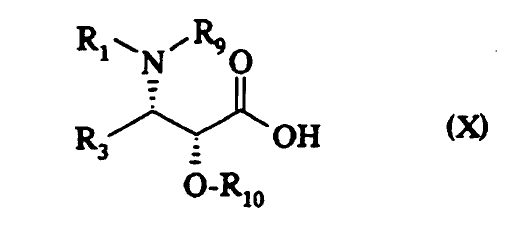 in which, either R₉ represents a hydrogen atom and R₁₀ represents a group protecting the hydroxyl function, or R₉ and R₁₀ together form a saturated 5- or 6-membered heterocycle, or by means of a derivative of this acid, and the protective groups are then replaced by hydrogen atoms, working under known conditions.

13. Pharmaceutical composition, **characterized in that** it contains at least one product according to one of Claims 1, 2 or 3 for which Z represents a radical of general formula (II) in combination with one or more pharmaceutically acceptable diluents or adjuvants and optionally one or more compatible and pharmacologically active compounds.
